# EUROPEAN PATENT SPECIFICATION

(11) **EP 4 007 561 B1**
(45) Date of publication and mention of the grant of the patent: **27.08.2025**
(21) Application number: 20746656.6
(22) Date of filing: 30.07.2020
(51) Int. Cl.: A61K 8/49, A61P 17/12, A61Q 19/00, A61K 31/505

(54) **PREVENTION AND REDUCTION OF CORNIFICATION DISORDERS AND RELATED COSMETIC AGENTS**
VORBEUGUNG UND REDUKTION VON VERHORNUNGSSTÖRUNGEN UND ZUGEHÖRIGE KOSMETISCHE MITTEL
PRÉVENTION ET RÉDUCTION DES TROUBLES DE LA KÉRATINISATION, ET AGENTS COSMÉTIQUES ASSOCIÉS

(30) Priority: 01.08.2019 EP 19189606
(43) Date of publication of application: 08.06.2022
(73) Proprietor: Merck Patent GmbH, 64293 Darmstadt (DE)
(72) Inventor: VON HAGEN, Joerg, 64319 PFUNGSTADT (DE); BICARD BENHAMOU, Valerie, 64297 DARMSTADT (DE)
(74) Representative: Merck Patent Association
(86) International application number: PCT/EP2020/071458
(87) International publication number: WO 2021/018990

(56) References cited:
- WO-A1-2010/043346
- WO-A1-2012/115247
- DE-U1- 202007 004 981
- US-A1- 2015 190 336
- US-A1- 2018 353 505

## Description

The present invention is directed to ectoin for use for improving skin conditions of keratosis pilaris of a mammal.

The stratum corneum is the outermost layer of the epidermis, consisting of dead cells (corneocytes). The stratum corneum is composed of 15 to 20 layers of flattened cells with no nuclei and cell organelles. Their cytoplasm shows filamentous keratin. These corneocytes are embedded in a lipid matrix composed of ceramides, cholesterol, cholesterol esthers and fatty acids.

The stratum corneum forms a barrier to protect underlying tissue from infection, dehydration, chemicals and mechanical stress. Desquamation, the process of cell shedding from the surface of the stratum corneum, balances proliferating keratinocytes from the stratum basale. These cells migrate through the epidermis towards the surface in a journey that takes approximately 14 days.

Cornification is the process of forming an epidermal barrier in stratified squamous epithelial tissue. During this process epidermal keratinocytes undergo terminal differentiation and programmed cell death. This results in the formation of a cornified skin layer, as well as in the formation of hair and nails.

Cornification is characterized by the replacement of intracellular organelles and content by a compact proteinaceous cytoskeleton, the cross-linking of proteins at the cell periphery to form a cornified cell envelope, and the linkage of corneocytes into a multicellular, functional but biologically dead structure. There are several variants of cornification leading to the different cornified structures.

The epidermis consists of several layers of different stages of keratinocyte differentiation. The cells of the basal layer are attached to the basement membrane by hemidesmosomes, have the capacity to proliferate and provide new cells that will differentiate towards the surface of the skin. Cells of the spinous layer no longer divide. In the granular layer, keratohyalin granules are present and proteins of the epidermal differentiation complex (EDC) are expressed. Filaggrin, also encoded in the EDC, is the main component of keratohyalin granules. Upon dephosphorylation and proteolysis of the profilaggrin precursor, filaggrin is dispersed and causes the aggregation of the keratin intermediate filaments. Several events then trigger the transition to the cornified layer, such as filaggrin degradation and cross-linking of keratins by transglutaminases. At the cytoplasmic side of the plasma membrane cross-linking of proteins forms the cornified envelope that is tightly connected via corneodesmosomes. The skin barrier is formed by tight junctions, which form strong intercellular interactions, and the lipids in the intercorneocyte spaces. Finally, the corneodesmosomes are proteolytically degraded by extracellular enzymes and the corneocytes are desquamated (see Eckhart et al, 2013, Biochimica et Biophysica Acta 1833, 3471-3480).

Disorders in the cornification process may result in various cosmetic problems affecting the visual appearance of the skin or may even result in dermatological problems, such as ichthyosis.

The object of the present invention is therefore the provision of active substances which can be used for improving skin conditions of keratosis pilaris of a mammal.

Surprisingly, it was now found that ectoine can effectively be used for such purpose.

Cornification disorders in the meaning of this application are disorders in the cornification process as described before.

Carbonylation in the meaning of this application includes protein oxidation. The use of compatible solutes, in particular ectoine and hydroxyectoine, for various cosmetic and pharmaceutical purposes is already known.

For example, WO 94/15923 describes that (S)-1,4,5,6-tetrahydro-2-methyl-4-pyrimidinecarboxylic acid or (S,S)-1,4,5,6-tetrahydro-5-hydroxy-2-methyl-4-pyrimidinecarboxylic acid can be used for the preparation of a cosmetic composition or a medicament, for example for the treatment of skin diseases. Furthermore, DE4342560 describes the use of ectoine and ectoine derivatives as moisturisers in cosmetic products. These products are suitable, for example, for the care of aged, dry or irritated skin. Furthermore, DE19933466 describes that ectoine and derivatives, such as hydroxyectoine, can be employed as free-radical scavengers in cosmetic and dermatological compositions. The compositions can be used for the treatment and/or prophylaxis of skin ageing caused by oxidative stress and of inflammatory reactions.

Further applications of ectoine and ectoine derivatives in cosmetic formulations are described, for example, in WO 00/07558, WO 00/07559, WO 00/07560, and US 7981899, such as, for example, the care and prophylaxis of dry and/or flaky skin, protection of the human skin against dryness and/or high salt concentrations, protection of cells, proteins and/or biomembranes of the human skin, protection of the microflora of the human skin, stabilisation of the skin barrier and protection and stabilisation of nucleic acids of human skin cells.

JP2002302444 discloses the use of formulations comprising ectoine for restoring the expression of the filaggrin gene caused by skin dryness. However, it was hitherto not known that these compounds, are advantageously suitable for preventing and reducing cornification disorders of the skin and/or the keratinized appendages to the skin, such as hair or nails.

From WO 2010/043346 the use of Antarcticine C (Pseudoalteromonas Ferment extract) for the treatment and/or care of conditions, disorders and/or pathologies associated with an alteration of the barrier function of the skin, scalp and/or nails is known. Keratosis pilaris is cited as one of the disorders and/or pathologies.

The present invention relates to ectoin for use for improving skin conditions of keratosis pilaris of a mammal.

In the biological context of organisms' production of gene products, downregulation is the process by which a cell decreases the quantity of a cellular component, such as a protein, in response to an external stimulus. The complementary process that involves increases of such components is called upregulation.

The above-mentioned non-therapeutic use of the compounds can take place *in-vitro* or *in-vivo.* The susceptibility of a particular cell to treatment with the compounds can be determined by testing *in vitro.* For testing *in vitro,* cultivated cells from a biopsy sample can be used.

The use of ectoine as defined above is typically topical.

According to a common definition compatible solutes are stress protection substances from extremely halophilic and halotolerant eubacteria, which accumulate them in large amounts through biosynthesis or effective transport mechanisms. These osmotically active substances prevent liquid outflow into the medium (drying out) and owe their name to the fact that they do not impair cell metabolism, even in high cytoplasmic concentration, i.e. are compatible with metabolism (according to: E.A. Galinski, M. Stein, B. Amendt, M. Kinder Comp. Biochem. Physiol., 117 (3) (1997) 357-365).

Ectoine and ectoine derivatives are low-molecular-weight, cyclic amino acid derivatives which can be isolated from various halophilic microorganisms or prepared synthetically. Both ectoine and hydroxyectoine have the advantage of not reacting with cell metabolism.

Ectoine refers to (S)-1,4,5,6-tetrahydro-2-methyl-4-pyrimidincarboxylic acid, hydroxyectoine to (S,S)-1,4,5,6-tetrahydro-5-hydroxy-2-methyl-4-pyrimidincarboxylic acid.

According to the present disclosure ectoine can be used as their physiologically tolerated salts and the stereoisomeric forms and can be present in the compositions in the form of optical isomers, diastereomers, racemates, zwitterions, cations or mixtures thereof.

Preferably, ectoine ((S)-1,4,5,6-tetrahydro-2-methyl-4-pyrimidincarboxylic acid) is used according to the present invention.

Preferred physiologically tolerated salts of the compounds are, for example, alkali metal, alkaline earth metal or ammonium salts, such as Na, K, Mg or Ca salts, and salts derived from the organic bases triethylamine or tris(2-hydroxyethyl)amine. Further preferred physiologically tolerated salts of the compounds arise through reaction with inorganic acids, such as hydrochloric acid, sulfuric acid and phosphoric acid, or with organic carboxylic or sulfonic acids, such as acetic acid, citric acid, benzoic acid, maleic acid, fumaric acid, tartaric acid and p-toluenesulfonic acid.

The preparation of the compounds is described in the literature (DE4342560). (S)-1,4,5,6-Tetrahydro-2-methyl-4-pyrimidinecarboxylic acid or (S,S)-1,4,5,6-tetrahydro-5-hydroxy-2-methyl-4-pyrimidinecarboxylic acid can also be obtained by microbiological methods (Severin et al., J. Gen. Microb. 138 (1992) 1629-1638 or EP1409707A).

The term mammal relates to vertebrate animals and humans, preferably to humans.

The keratinized appendages include the nails and hair of humans, and the horns, hoofs, claws and fur of animals. In the living mammal, such keratinized appendages are subjected to countless traumas from the environment to which they are exposed during everyday movements. In humans, the hardness and strength of the nails, which includes fingernails and toenails, is particularly important not only for the beauty of their appearance, but for the well-being of the individual. Embrittlement of the nails is normally associated with aging. However, various activities expose the nails to a number of materials which also adversely affect the nail's physico-mechanical condition, such as exposure to soaps, detergents, manicuring. Additionally, certain diseases of widely different causes and symptoms can also lead to nail embrittlement or associated disfigurement owing to weakening of nail hardness and strength.

Cornification disorders can be prevented and/or reduced by protecting the regular concentrations of proteins involved in cornification, and/or up- and/or downregulating such proteins.

For the functionality of the skin barrier, mainly the top layers of the stratum corneum on the one hand need to keep up the constant renewal of proteins and lipids in the required balance and on the other hand the proteins and lipids produced during the cornification process need to stay active and assure the functionality and avoid inactivation by degradation or events like carbonylation ideally until desquamation . Both effects increase an intact skin barrier function of the stratum corneum.

The present disclosure relates to the upregulation and/or reduction of the carbonylation of one or more of the proteins selected from the group consisting of filaggrin, hornerin and LAMP2a and/or the reduction of the carbonylation of the protein filaggrin-2.

In particular, the present disclosure relates to the upregulation of one or more of the proteins selected from the group consisting of filaggrin, hornerin and LAMP2a.

In particular, the present disclosure relates to the reduction of the carbonylation of one or more of the proteins selected from the group consisting of filaggrin, hornerin, LAMP2a and/or filaggrin-2.

Filaggrin (filament aggregating protein) is a filament-associated protein that binds to keratin fibers in epithelial cells. Ten to twelve filaggrin units are post-translationally hydrolyzed from a large profilaggrin precursor protein (350kDa) during terminal differentiation of epidermal cells. In humans profilaggrin is encoded by the FLG gene, which is part of the S100 fused-type protein (SFTP) family within the epidermal differentiation complex on chromosome 1q21. Disorders in filaggrin expression are strongly predisposed to a severe form of dry skin, ichthyosis vulgaris or eczema.

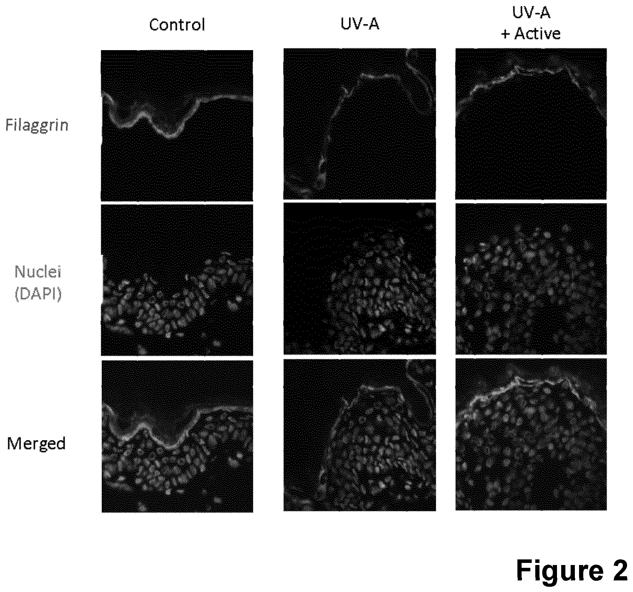

Filaggrin-2 is encoded by the gene *FLG2.* The protein is essential for normal cell-cell adhesion in the cornified layers and important for proper integrity and mechanical strength of the stratum corneum of the epidermis.

It was shown that filaggrin-2 is important for a proper cornification and a functional stratum corneum. Patients with atopic dermatitis show a decreased expression of filaggrin-2 (Pendaries et al, 2015, Cell Death and Disease 6, page e1656).

It is shown that ectoine reduces the carbonylation of this protein required for cell-cell junction interaction. Carbonylation might result in loss of function which in turn would have a negative impact on the connectivity of the keratinocytes and thus reduces the barrier function.

Hornerin is a protein having 2496 amino acids and comprising EF-hand domains at the N-terminus followed by a spacer sequence and a large repetitive domain. It was found that hornerin is expressed in such tissues which are cornifying stratified epithelium. Hornerin was also detected in the granular and cornified layers of the mature epidermis (Makino et al, 2001, The Journal of Biological Chemistry 276, 47445-47452). This publication further suggests that the close similarity of hornerin to profilaggrin indicates that hornerin is associated with some hereditary ichthyotic disorders.

LAMP2A (Lysosome-associated membrane protein 2A) is one of the lysosome-associated membrane glycoproteins and is the receptor for chaperone-mediated autophagy. The protein has 410 amino acids and is X-chromosome associated. In skin it is known to be expressed moderately in all skin cell types. LAMP2A is involved in the autophagy and cell renewal process and this impairing the skin barrier built up. Epidermal keratinocyte nuclear removal (nucleophagy) is a crucial step in the cornification process. In this process some of the nuclear content is removed by the autophagolysosome, a LC3-positive/LAMP2-positive body (Rogerson et al, 2018, Nucleus 9, 56-64).

Within this regard it is found that ectoine and/or hydroxyectoine influence the presence of filaggrin, filaggrin-2, LAMP2a and hornerin, resulting in a positive influence on the cornification process. Ectoine reduces the carbonylation of filaggrin-2 which results in a faster cornification. Ectoine also reduces the carbonylation of LAMP2a, maintaining the autophagy (nucleophagy) during the cornification process. The effect of ectoine on filaggrin and hornerin is mainly to keep the functionality of both proteins, as carbonylation might result in loss of function of both proteins, which means that these proteins are no longer able to perform their crucial task in the cornification process, since it is known that proteins aggregate as a result of carbonylation hindering cells in multiple ways. Cells are stressed and the aggregates need to be recycled over the ubiquitination-proteasome pathway, resulting also in increased autophagy. In both cases cells are distracted to a certain extend to fulfill their main objective to build up proteins for the stratum corneum. The result is an impairment of the skin barrier function. ectoine will reduce both signs by lowering the inactivation of fillaggrin and hornerin.

Therefore, the present disclosure further relates to a non-therapeutic use of ectoine and/or hydroxyectoine as cosmetic agent which reduces the carbonylation of one or more of the proteins selected from the group consisting of filaggrin, hornerin, LAMP2a and/or filaggrin-2 of the skin and/or the keratinized appendages to the skin in a mammal, preferably a human.

Therefore, the present disclosure further relates to an non-therapeutic use of ectoine and/or hydroxyectoine as cosmetic agent which upregulates one or more of the proteins selected from the group consisting of filaggrin, hornerin and LAMP2a of the skin and/or the keratinized appendages to the skin in a mammal, preferably a human.

Therefore, the present disclosure further relates to ectoine and/or hydroxyextoine for use in preventing and/ or reducing cornification disorders of the skin and/ or the keratinized appendages to the skin in a mammal, characterized in that it involves the reduction of the carbonylation of one or more of the proteins selected from the group consisting of filaggrin, hornerin, LAMP2a and/or filaggrin-2.

Therefore, the present disclosure further relates to ectoine and/or hydroxyextoine for use in preventing and/ or reducing cornification disorders of the skin and/ or the keratinized appendages to the skin in a mammal, characterized in that it involves the upregulation of one or more of the proteins selected from the group consisting of filaggrin, hornerin and LAMP2a.

In a further aspect the present disclosure relates to a formulation for the use in the treatment of cornification disorders comprising ectoine/hydroxyectoine. Such formulations can be prepared within common knowledge of a person skilled in the art.

In a further aspect the present disclosure relates to a formulation for the use in the treatment of cornification disorders comprising ectoine/hydroxyectoine, characterized in that the cornification disorder shows itself in ichthyosis and/ or atopic skin.

Similarly, cosmetic formulations comprising the active components can be prepared.

In a further aspect the present disclosure relates to a cosmetic formulation comprising ectoine and/or hydroxyectoine as cosmetic agent which reduces the carbonylation of one or more of the proteins selected from the group consisting of filaggrin, hornerin, LAMP2a and/or filaggrin-2 of the skin and/or the keratinized appendages to the skin in a mammal.

In a further aspect the present disclosure relates to a cosmetic formulation comprising ectoine and/or hydroxyectoine as cosmetic agent which upregulates one or more of the proteins selected from the group consisting of filaggrin, hornerin and LAMP2a of the skin and/or the keratinized appendages to the skin in a mammal.

For the purposes of the present invention, the term "composition" or "formulation" is also used synonymously alongside the term "preparation".

The preparations here are usually preparations which can be applied topically, such as, for example, cosmetic or dermatological formulations or medical products. "Can be applied topically" in the sense of the invention means that the preparation is applied externally and locally, i.e. that the preparation must be suitable, for example, for being able to be applied to the skin or hair. In this case, the preparations comprise a cosmetically, pharmaceutically or dermatologically suitable vehicle and, depending on the desired property profile, optionally further suitable ingredients. The topical preparations are preferably employed as cosmetic or dermatological preparation, particularly preferably as cosmetic preparation. Suitable vehicles and assistants or fillers are described in detail in the following part.

The preparations may include or comprise, essentially consist of or consist of the necessary or optional constituents mentioned above and/or below. All compounds or components which can be used in the preparations are either known and commercially available or can be synthesised by known processes.

Ectoine or hydroxyectoine, as indicated above and also as preferably described, is typically present in the preparation in an amount of 0.001 to 50% by weight, based on the total weight of the preparation, preferably from 0.01 to 10% by weight and especially preferably from 0.1 to 10% by weight, based on the composition as a whole. The proportion of the said compounds in the composition is very especially preferably from 0.1 to 5% by weight, based on the composition as a whole. The person skilled in the art is presented with absolutely no difficulties in selecting appropriately the amounts depending on the intended effect of the preparation.

Besides ectoine and/or hydroxyectoine, the compositions may also comprise further cosmetic, dermatological or pharmaceutical active ingredients.

Further active compounds are preferably selected from the group of UV filters, pore-refining agents, antioxidants, vitamins, skin-lightening active compounds, anti-ageing active compounds, anti-inflammatory active compounds, antimicrobial active compounds, active compounds for improving the moisture content of the skin (skin-moisture regulators), anticellulite active compounds, antiwrinkle active compounds, antidandruff active compounds, anti-acne active compounds, deodorants, pigments and self-tanning substances; particularly preferably from the group of UV filters, pore-refining agents, antioxidants, vitamins, skin-lightening active compounds, self-tanning substances, anti-ageing active compounds and anticellulite active compounds.

In one preferred embodiment the formulation further comprises UV filters. In principle, all UV filters are suitable for combination within the preparation according to the invention. Particular preference is given to UV filters whose physiological acceptability has already been demonstrated. There are many proven substances known from the specialist literature both for UVA and also UVB filters. The compounds shown in the following lists should only be regarded as examples. Other UV filters can of course also be used.

Preferred preparations may comprise organic UV filters, so-called hydrophilic or lipophilic sun-protection filters, which are effective in the UVA region and/or UVB region and/or IR and/or VIS region (absorbers). These substances can be selected, in particular, from dibenzoylmethane derivatives, p-aminobenzoic acid derivatives, salicylic acid derivatives, β,β-diphenylacrylate derivatives, camphor derivatives, triazine derivatives, cinnamic acid derivatives and polymeric filters and silicone filters, which are described in the application WO 93/04665. Further examples of organic filters are indicated in the patent application EP-A 0 487 404. The said UV filters are usually named below in accordance with INCI nomenclature.

Particularly suitable for a combination are:
Dibenzoylmethane derivatives: 4-isopropyl-dibenzoyl-methane and 4,4'-methoxy-tert-butyl-dibenzoylmethane being described in FR-A-2326405, FR-A-2440933 and EP-A-0114607. 4,4'-Methoxy-tert-butyl-dibenzoylmethane is for example marketed by Merck under the name "Eusolex 9020".

para-Aminobenzoic acid and derivatives thereof: PABA, Ethyl PABA, Ethyl dihydroxypropyl PABA, Ethylhexyl dimethyl PABA, for example marketed by ISP under the name "Escalol 507", Glyceryl PABA, PEG-25 PABA, for example marketed by BASF under the name "Uvinul P25".

Salicylates: Homosalate marketed by Merck under the name "Eusolex HMS"; Ethylhexyl salicylate, for example marketed by Symrise under the name "Neo Heliopan OS"; Dipropylene glycol salicylate, for example marketed by Scher under the name "Dipsal"; TEA salicylate, for example marketed by Symrise under the name "Neo Heliopan TS".

β,β-Diphenylacrylate derivatives: Octocrylene, for example marketed by Merck under the name "Eusolex^{®} OCR"; "Uvinul N539" from BASF; Etocrylene, for example marketed by BASF under the name "Uvinul N35".

Benzophenone derivatives: benzophenone-1, for example marketed under the name "Uvinul 400"; benzophenone-2, for example marketed under the name "Uvinul D50"; benzophenone-3 or oxybenzone, for example marketed under the name "Uvinul M40"; benzophenone-4, for example marketed under the name "Uvinul MS40"; benzophenone-9, for example marketed by BASF under the name "Uvinul DS-49"; benzophenone-5, benzophenone-6, for example marketed by Norquay under the name "Helisorb 11"; benzophenone-8, for example marketed by American Cyanamid under the name "Spectra-Sorb UV-24"; benzophenone-12 n-hexyl 2-(4-diethylamino-2-hydroxybenzoyl) benzoate or 2-hydroxy-4-methoxybenzophenone, marketed by Merck, Darmstadt, under the name Eusolex^{®} 4360.

Benzylidenecamphor derivatives: 3-benzylidenecamphor, for example marketed by Chimex under the name "Mexoryl SD"; 4-methylbenzylidenecamphor, for example marketed by Merck under the name "Eusolex 6300"; benzylidenecamphorsulfonic acid, for example marketed by Chimex under the name "Mexoryl SL"; Camphor benzalkonium methosulfate, for example marketed by Chimex under the name "Mexoryl SO"; terephthalylidene-dicamphorsulfonic acid, for example marketed by Chimex under the name "Mexoryl SX"; polyacrylamidomethylbenzylidenecamphor marketed by Chimex under the name "Mexoryl SW'.

Phenylbenzimidazole derivatives: phenylbenzimidazolesulfonic acid, for example marketed by Merck under the name "Eusolex 232"; disodium phenyl dibenzimidazole tetrasulfonate, for example marketed by Symrise under the name "Neo Heliopan AP".

Phenylbenzotriazole derivatives: Drometrizol trisiloxane, for example marketed by Rhodia Chimie under the name "Silatrizole"; Methylenebis(benzotriazolyl)tetramethylbutylphenol in solid form, for example marketed by Fairmount Chemical under the name "MIXXIM BB/100", or in micronised form as an aqueous dispersion, for example marketed by BASF under the name "Tinosorb M".

Triazine derivatives: Ethylhexyltriazone, for example marketed by BASF under the name "Uvinul T150"; Diethylhexylbutamidotriazone, for example marketed by Sigma 3V under the name "Uvasorb HEB"; 2,4,6-tris-(diisobutyl 4'-aminobenzalmalonate)-s-triazine or 2,4,6-Tris(biphenyl)-1,3,5-triazine marketed by BASF as Tinosorb A2B; 2,2'-[6-(4-methoxyphenyl)-1,3,5-triazine-2,4-diyl]bis[5-(2-ethylhexyl)oxy]phenol; marketed by BASF as Tinosorb S; N2,N4-bis[4-[5-(1,1-dimethylpropyl)-2-benzoxazolyl]phenyl]-N6-(2-ethylhexyl)-1,3,5-triazine-2,4,6-triamine marketed as Uvasorb K 2A by Sigma 3V, or trisbiphenyltriazin, marketed as Tinosorb^{®} A2B by BASF.

Anthraniline derivatives: Menthyl anthranilate, for example marketed by Symrise under the name "Neo Heliopan MA".

Imidazole derivatives: ethylhexyldimethoxybenzylidenedioxoimidazoline propionate.

Benzalmalonate derivatives: polyorganosiloxanes containing functional benzalmalonate groups, such as, for example, Polysilicone-15, for example marketed by Hoffmann LaRoche under the name "Parsol SLX".

4,4-Diarylbutadiene derivatives: 1,1-Dicarboxy(2,2'-dimethylpropyl)-4,4-diphenylbutadiene.

Benzoxazole derivatives: 2,4-bis[5-(1-dimethylpropyl)benzoxazol-2-yl(4-phenyl) imino]-6-(2-ethylhexyl)imino-1,3,5-triazine, for example marketed by Sigma 3V under the name Uvasorb K2A, and mixtures comprising this.

Piperazine derivatives, such as, for example, the compound 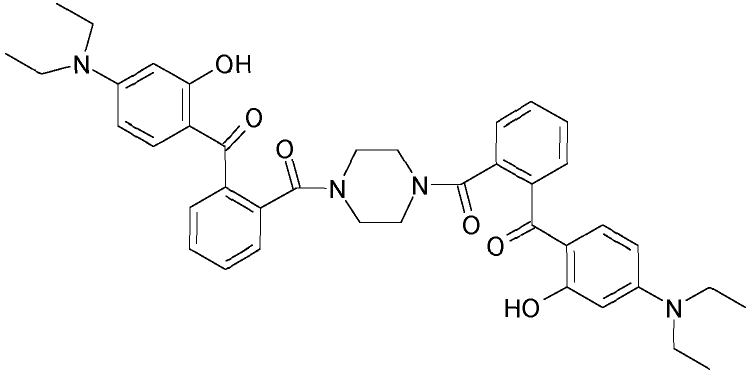 or the UV filters of the following structures

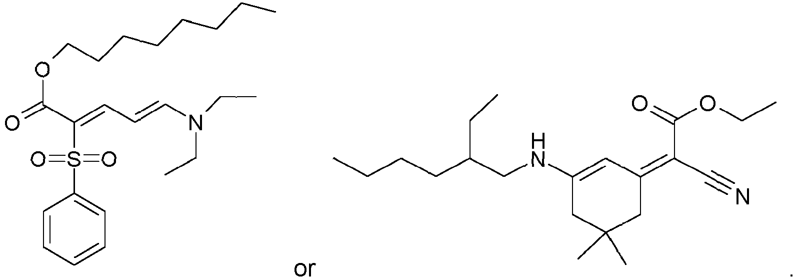

It is also possible to use UV filters based on polysiloxane copolymers having a random distribution in accordance with the following formula, where, for example, a = 1,2; b= 58 and c=2,8:

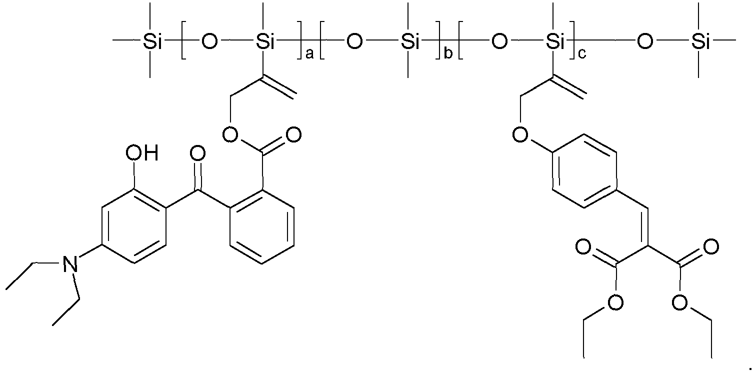

This list of compounds represents examples; it is of course also possible to use other UV filters.

Suitable organic UV-protecting substances can preferably be selected from the following list: Ethylhexyl salicylate, phenylbenzimidazolesulfonic acid, benzophenone-3, benzophenone-4, benzophenone-5, n-Hexyl 2-(4-diethyl-amino-2-hydroxybenzoyl)benzoate, 4-methylbenzylidenecamphor, tere-phthalylidenedicamphorsulfonic acid, disodium phenyldibenzimidazole-tetrasulfonate, methylenebis(benzotriazolyl)tetramethylbutylphenol, Butyl methoxydibenzoylmethane, Ethylhexyltriazone, Diethylhexylbutamidotriazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine and mixtures thereof.

These organic UV filters are generally incorporated into formulations in an amount of 0.01 to 20 per cent by weight, preferably 1 to 10% by weight.

The preparations may comprise further inorganic UV filters, so-called particulate UV filters. These combinations with particulate UV filters are possible both as powder and also as dispersion or paste of the following types.

Preference is given here both to those from the group of the titanium dioxides, such as, for example, coated titanium dioxide (for example Eusolex^{®} T-2000, Eusolex^{®} T-AQUA, Eusolex^{®} T-AVO, Eusolex^{®} T-PRO, Eusolex^{®} T-EASY), zinc oxides (for example Sachtotec^{®}), iron oxides or also cerium oxides and/or zirconium oxides.

Furthermore, combinations with pigmentary titanium dioxide or zinc oxide are also possible, where the particle size of these pigments is greater than or equal to 200 nm, for example Hombitan^{®} FG or Hombitan^{®} FF-Pharma.

It may furthermore be preferred for the preparations to comprise inorganic UV filters which have been aftertreated by conventional methods, as described, for example, in Cosmetics & Toiletries 1990, 105, 53. One or more of the following aftertreatment components can be selected here: amino acids, beeswax, fatty acids, fatty acid alcohols, anionic surfactants, lecithin, phospholipids, sodium, potassium, zinc, iron or aluminium salts of fatty acids, polyethylenes, silicones, proteins (particularly collagen or elastin), alkanolamines, silicon dioxide, aluminium oxide, further metal oxides, phosphates, such as sodium hexametaphosphate, or glycerine.

Particulate UV filters which are preferably employed here are:
- untreated titanium dioxides, such as, for example, the products Microtitanium Dioxide MT 500 B from Tayca; titanium dioxide P25 from Degussa;
- Aftertreated micronised titanium dioxides with aluminium oxide and silicon dioxide aftertreatment, such as, for example, the product "Microtitanium Dioxide MT 100 SA from Tayca, or the product "Tioveil Fin" from Uniqema;
- Aftertreated micronised titanium dioxides with aluminium oxide and/or aluminium stearate/laurate aftertreatment, such as, for example, Microtitanium Dioxide MT 100 T from Tayca; Eusolex T-2000 from Merck;
- Aftertreated micronised titanium dioxides with iron oxide and/or iron stearate aftertreatment, such as, for example, the product "Microtitanium Dioxide MT 100 F" from Tayca;
- Aftertreated micronised titanium dioxides with silicon dioxide, aluminium oxide and silicone aftertreatment, such as, for example, the product "Microtitanium Dioxide MT 100 SAS", from Tayca;
- Aftertreated micronised titanium dioxides with sodium hexametaphosphate, such as, for example, the product "Microtitanium Dioxide MT 150 W' from Tayca.

The treated micronised titanium dioxides employed for the combination may also be aftertreated with:
- Octyltrimethoxysilanes, such as, for example, the product Tego Sun T 805 from Degussa;
- Silicon dioxide; such as, for example, the product Parsol T-X from DSM;
- Aluminium oxide and stearic acid; such as, for example, the product UV-Titan M160 from Sachtleben;
- Aluminium and glycerine; such as, for example, the product UV-Titan from Sachtleben,
- Aluminium and silicone oils, such as, for example, the product UV-Titan M262 from Sachtleben;
- Sodium hexamethaphosphate and polyvinylpyrrolidone,
- Polydimethylsiloxanes, such as, for example, the product 70250 Cardre UF TiO2SI3" from Cardre;
- Polydimethylhydrogenosiloxanes, such as, for example, the product Microtitanium Dioxide USP Grade Hydrophobic" from Color Techniques.

The combination with the following products may furthermore also be advantageous:
- Untreated zinc oxides, such as, for example, the product Z-Cote from BASF (Sunsmart), Nanox from Elementis;
- Aftertreated zinc oxides, such as, for example, the following products:
   - "Zinc Oxide CS-5" from Toshibi (ZnO aftertreated with polymethyl-hydrogenosiloxane);
   - Nanogard Zinc Oxide FN from Nanophase Technologies;
   - "SPD-Z1" from Shin-Etsu (ZnO aftertreated with a silicone-grafted acrylic polymer, dispersed in cyclodimethylsiloxanes);
   - "Escalol Z100" from ISP (aluminium oxide-aftertreated ZnO, dispersed in an ethylhexyl methoxycinnamate/PVP-hexadecene/ methicone copolymer mixture);
   - "Fuji ZNO-SMS-10" from Fuji Pigment (ZnO aftertreated with silicon dioxide and polymethylsilesquioxane);
   - Untreated cerium oxide micropigment, for example with the name "Colloidal Cerium Oxide" from Rhone Poulenc;
   - Untreated and/or aftertreated iron oxides with the name Nanogar from Arnaud.

By way of example, it is also possible to employ mixtures of various metal oxides, such as, for example, titanium dioxide and cerium oxide, with and without aftertreatment, such as, for example, the product Sunveil A from Ikeda. In addition, mixtures of aluminium oxide-, silicon dioxide- and silicone-aftertreated titanium dioxide/zinc oxide mixtures, such as, for example, the product UV-Titan M261 from Sachtleben, can also be employed.

These inorganic UV filters are generally incorporated into the preparations in an amount of 0.1 to 25 per cent by weight, preferably 2 to 10% by weight. By combination of one or more of the said compounds having a UV filter action, the protective action against harmful effects of the UV radiation can be optimised.

All said UV filters can also be employed in encapsulated form. In particular, it is advantageous to employ organic UV filters in encapsulated form. The capsules in preparations to be employed in accordance with the invention are preferably present in amounts which ensure that the encapsulated UV filters are present in the preparation in the per cent by weight ratios indicated above.

The formulation may preferably further comprise a pore-refining agent. Pore refining agents are, for example, retinol (vitamin A), 5,7-dihydroxy-2-methylchromone, marketed under the trade name RonaCare^{®} Luremine, nicotinamide or isoquercetin.

In a further preferred embodiment the formulation further comprises at least one skin-lightening active compound (or synonymously depigmentation active compounds) or extracts having a skin-lightening activity.

Skin-lightening active compounds can in principle be all active compounds known to the person skilled in the art. Suitable for combination are commercially available melanogenesis inhibitors, such as, for example, ascorbic acid and derivatives thereof, aloesin, niacinamide, emblica, elagic acid, liquorice extract, mulberry extract, kojic acid, liquorice extract, rucinol, hydroquinone, azelaic acid, arbutin, magnesium ascorbyl phosphate, lactic acid, butylphenylmethoxyphenylpropandiol (available as RonaCare^{®} PristineBright^{®} from Merck) or the like. Preferred examples of compounds having skin-lightening activity are hydroquinone, niacinamide, ascorbic acid and physiologically acceptable salts thereof, kojic acid, arbutin, aloesin, azelaic acid, elagic acid, lactic acid, butylphenylmethoxyphenylpropandiol or rucinol. Preferred examples of extracts having skin-lightening activity are liquorice extract, mulberry extract or emblica.

The preparation as described above may further comprise one or more self-tanning substances. A preparation of this type generally has a contrast-reduction effect and enables a uniform skin shade to be achieved. The invention likewise relates to the use of compatible solutes, as described, in combination with one or more self-tanning substances for contrast reduction and achieving a uniform skin shade. A contrast-reduction agent is accordingly a substance which reduces a non-uniform skin coloration by reducing the contrast between more strongly and less strongly coloured skin areas. A uneven skin coloration of this type can arise here through non-uniform pigmentation and/or a different distribution of the horny skin. Uneven pigmentation is by no means unusual in the population and is based on different levels of melanin production by the melanocytes or an irregular distribution of the melanocytes in the skin.

A contrast reduction can be achieved, in particular, by preparations in which a self-tanning substance is further present. Such self-tanning substances may be self-tanning substances reacting with the amino acids of the skin based on a Maillard reaction or Michael addition, or may be so-called melanogenesis-promoting substances or propigmentation substances, which promote the natural pigmentation of the skin.

Preferred self-tanning substances are, for example: 1,3-dihydroxyacetone (DHA) and derivatives derived therefrom, glycerolaldehyde, hydroxymethylglyoxal, γ-dialdehyde, erythrulose, 6-aldo-D-fructose, ninhydrin, 5-hydroxy-1,4-naphtoquinone (juglone) or 2-hydroxy-1,4-naphtoquinone (lawsone) or a mixture of the said compounds. Particularly preferred is 1,3-dihydroxyacetone, erythrulose and their mixture.

Propigmentation substances are known to the person skilled in the art. Examples are glycerrithinic acid, melanocyte-activating hormone (alpha-MSH), peptide analoga, thymidine-dinucleotide, L-tyrosine and their esters, bicyclic monoterpendiole (described in Brown et al., Photochemistry and Photobiology B: Biology 63 (2001) 148-161) or 7-acyloxy-chromen-4-on-derviatives (described in WO 2012/097857 A1), in particular hexadecanic acid 5-hydroxy-2-methyl-4-oxo-4H-chromen-7-yl ester (available from Merck KGaA, Darmstadt, Germany under Ronacare^{®} Bronzyl^{™}).

Preferably the self-tanning substance is present in the composition in an amount of 0,01 to 20 % by weight, more preferably in an amount of 0,5 to 15 % by weight and most preferably in an amount of 1 to 8 % by weight, related to the complete weight of the preparation.

In the preparations described coloured pigments may furthermore also be present, where the layer structure of the pigments is not limited. On use of 0.5 to 5% by weight, the coloured pigment should preferably be skin-coloured or brownish. The selection of a corresponding pigment is familiar to the person skilled in the art.

In a further preferred embodiment of the formulation, the preparation comprises one or more antioxidants and/or one or more vitamins. The use of antioxidants enables a protective action against oxidative stress or against the effect of free radicals in general to be achieved, the person skilled in the art being presented with absolutely no difficulties in selecting antioxidants which act suitably quickly or with a time delay. There are many proven substances known from the specialist literature which can be used as antioxidants, for example amino acids (for example glycine, histidine, tyrosine, tryptophan) and derivatives thereof, imidazoles (for example urocanic acid) and derivatives thereof, peptides, such as, for example, D,L-carnosine, D-carnosine, L-carnosine and derivatives thereof (for example anserine), carotinoids, carotenes (such as, for example, α-carotene, β-carotene, lyco-pene) and derivatives thereof, chlorogenic acid and derivatives thereof, lipoic acid and derivatives thereof (such as, for example, dihydrolipoic acid), aurothioglucose, propylthiouracil and other thiols (such as, for example, thioredoxin, glutathione, cysteine, cystine, cystamine and the glycosyl, N-acetyl, methyl, ethyl, propyl, amyl, butyl and lauryl, palmitoyl, oleyl, γ-lino-leyl, cholesteryl and glyceryl esters thereof) and salts thereof, dilauryl thiodipropionate, distearyl thiodipropionate, thiodipropionic acid and derivatives thereof (such as, for example, esters, ethers, peptides, lipids, nucleotides, nucleosides and salts), and sulfoximine compounds (such as, for example, buthionine sulfoximines, homocysta sulfoximine, buthionine sulfones, penta-, hexa- and heptathionine sulfoximine) in very low tolerated doses (such as, for example, pmol to µmol/kg), and also (metal) chelating agents, (such as, for example, α-hydroxyfatty acids, palmitic acid, phytic acid, lactoferrin), α-hydroxy acids (such as, for example, citric acid, lactic acid, malic acid), humic acid, bile acid, bile extracts, bilirubin, biliverdin, EDTA, EGTA, pentasodium ethylenediamine tetramethylene phosphonate and derivatives thereof, unsaturated fatty acids and derivatives thereof, vitamin C and derivatives (such as, for example, ascorbyl palmitate, magnesium ascorbyl phosphate, ascorbyl acetate), tocopherols and derivatives (such as, for example, vitamin E acetate), vitamin A and derivatives (such as, for example, vitamin A palmitate) and coniferyl benzoate of benzoin resin, rutinic acid and derivatives thereof, α-glycosylrutin, ferulic acid, furfuryli-deneglucitol, carnosine, butylhydroxytoluene, butylhydroxyanisole, nor-dihydroguaiaretic acid, trihydroxybutyrophenone, quercetin, uric acid and derivatives thereof, mannose and derivatives thereof, zinc and derivatives thereof (such as, for example, ZnO, ZnSO₄), selenium and derivatives thereof (such as, for example, selenomethionine), stilbenes and derivatives thereof (such as, for example, stilbene oxide, trans-stilbene oxide). Further suitable antioxidants are also described in WO 2006/111233 and WO 2006/111234.

Suitable antioxidants are also compounds of the general formulae A or B 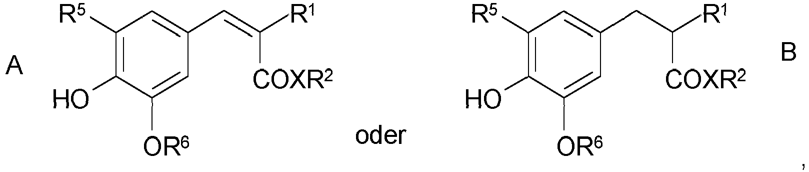 in which
- R¹: denotes -C(O)CH₃, -CO₂R³, -C(O)NH₂ and -C(O)N(R⁴)₂,
- X: denotes O or NH,
- R²: denotes linear or branched alkyl having 1 to 30 C atoms,
- R³: denotes linear or branched alkyl having 1 to 20 C atoms,
- R⁴: in each case, independently of one another, denotes H or linear or branched alkyl having 1 to 8 C atoms,
- R⁵: denotes H, linear or branched alkyl having 1 to 8 C atoms or linear or branched alkoxy having 1 to 8 C atoms, and
- R⁶: denotes linear or branched alkyl having 1 to 8 C atoms.

Preference is given to derivatives of 2-(4-hydroxy-3,5-dimethoxybenzylidene)malonic acid and/or 2-(4-hydroxy-3,5-dimethoxybenzyl)malonic acid, particularly preferably bis(2-ethylhexyl) 2-(4-hydroxy-3,5-dimethoxybenzylidene)malonate (for example Oxynex^{®} ST Liquid) and/or bis(2-ethylhexyl) 2-(4-hydroxy-3,5-dimethoxybenzy)malonate (for example RonaCare^{®} AP). Mixtures of antioxidants are likewise suitable for use in the preparations.

Known and commercial mixtures are, for example, mixtures comprising, as active ingredients, lecithin, L-(+)-ascorbyl palmitate and citric acid, natural tocopherols, L-(+)-ascorbyl palmitate, L-(+)-ascorbic acid and citric acid (such as, for example, Oxynex^{®} K LIQUID), tocopherol extracts from natural sources, L-(+)-ascorbyl palmitate, L-(+)-ascorbic acid and citric acid (such as, for example, Oxynex^{®} L LIQUID), DL-α-tocopherol, L-(+)-ascorbyl palmitate, citric acid and lecithin (such as, for example, Oxynex^{®} LM) or butylhydroxytoluene (BHT), L-(+)-ascorbyl palmitate and citric acid (such as, for example, Oxynex^{®} 2004). Antioxidants of this type are usually employed in such compositions with compounds of the formula (I) or part-formulae thereof in per cent by weight ratios in the range from 1000:1 to 1:1000, preferably in per cent by weight ratios of 100:1 to 1:100.

Of the phenols having an antioxidative action, the polyphenols, some of which are naturally occurring, are of particular interest for applications in the pharmaceutical, cosmetic or nutrition sector. For example, the flavonoids or bioflavonoids, which are principally known as plant dyes, frequently have an antioxidant potential. Lemanska et al., Current Topics in Biophysics 2000, 24(2), 101-108, are concerned with effects of the substitution pattern of mono- and dihydroxyflavones. It is observed therein that dihydroxyflavones containing an OH group adjacent to the keto function or OH groups in the 3'4'- or 6,7- or 7,8-position have antioxidative properties, while other mono- and dihydroxyflavones in some cases do not have antioxidative properties. Quercetin (cyanidanol, cyanidenolon 1522, meletin, sophoretin, ericin, 3,3',4',5,7-pentahydroxyflavone) is frequently mentioned as a particularly effective antioxidant (for example Rice-Evans et al., Trends in Plant Science 1997, 2(4), 152-159). Lemanska et al., Free Radical Biology & Medicine 2001, 31(7), 869-881, have investigated the pH dependence of the antioxidant action of hydroxyflavones. Quercetin exhibits the highest activity amongst the structures investigated over the entire pH range.

The preparations may comprise vitamins as further ingredients. Vitamins and vitamin derivatives selected from vitamin A, vitamin A propionate, vitamin A palmitate, vitamin A acetate, retinol, vitamin B, thiamine chloride hydrochloride (vitamin B₁), riboflavin (vitamin B₂), nicotinamide, vitamin C (ascorbic acid), vitamin D, ergocalciferol (vitamin D₂), vitamin E, DL-α-tocopherol, tocopherol E acetate, tocopherol hydro-gensuccinate, vitamin K1, esculin (vitamin P active compound), thiamine (vitamin B₁), nicotinic acid (niacin), pyridoxine, pyridoxal, pyridoxamine, (vitamin B₆), panthothenic acid, biotin, folic acid and cobalamine (vitamin B₁₂) are preferably present in the preparations according to the invention, particularly preferably vitamin A palmitate, vitamin C and derivatives thereof, DL-α-tocopherol, tocopherol E acetate, nicotinic acid, pantothenic acid and biotin. In the case of cosmetic application, vitamins are usually added with the preparations in ranges from 0.01 to 5% by weight, based on the total weight. Nutrition-physiological applications are oriented towards the respective recommended vitamin requirement.

The formulation may further comprise at least one substance which serves for maintaining and/or improving the moisture content of the skin. These substances can, without this being intended to be regarded as a restriction, also be, inter alia, substances which belong to the so-called natural moisturising factors, such as, for example, 2-oxopyrrolidine 5-carboxylic acid.

The preparations may in addition comprise anti-ageing active compounds, anticellulite active compounds or conventional skin-protecting or skin-care active compounds. Skin-protecting or skin-care active compounds can in principle be all active compounds known to the person skilled in the art. Particularly preferred anti-ageing active compounds are pyrimidinecarboxylic acids, aryl oximes, bioflavonoids, bio-flavonoid-containing extracts, chromones or retinoids.

Additionally, anti-ageing active compounds which can be used are products from Merck, such as, for example, 5,7-dihydroxy-2-methylchromone, marketed under the trade name RonaCare^{®} Luremine, Ronacare^{®} Isoquercetin, Ronacare^{®} Tilirosid or Ronacare^{®} Cyclopeptide 5.

Known anti-ageing substances are also chromones, as described, for example, in EP 1508327, or retinoids, for example retinol (vitamin A), reti-noic acid, retinaldehyde or also synthetically modified compounds of vitamin A. The chromones and retinoids described are simultaneously also effective anticellulite active compounds. An anticellulite active compound which is likewise known is caffeine.

In the above-mentioned formulations, the compatible solutes can advantageously be combined with all known preservatives or antimicrobial active compounds, such as, for example, anisic acid, alcohol, ammonium benzoate, ammonium propionate, benzoic acid, bronopol, butylparaben, benzethonium chloride, benzalkonium chloride, 5-bromo-5-nitro-1,3-dioxane, benzyl alcohol, boric acid, benzisothiazolinone, benzotriazole, benzyl hemiformate, benzylparaben, 2-bromo-2-nitropropane-1,3-diol, butyl benzoate, chlorphenesin, capryl/capric glycerides, caprylyl glycol, Camellia Sinensis leaf extract, Candida Bombicola/glucose/methyl rapeseedates, chloroxylenol, chloroacetamide, chlorhexidine, chlorobutanol, calcium benzoate, calcium paraben, calcium propionate, calcium salicylate, calcium sorbate, captan, chloramine T, chlorhexidine diacetate, chlorhexidine digluconate, chlorhexidine dithydrochloride, chloroacetamine, p-chloro-m-cresol, chlorphen, p-chlorophenol, chlorothymol, Citrus Grandis (grapefruit) fruit extract, Citrus Grandis (grapefruit) seed extract, m-cresol, o-cresol, p-cresol, mixed cresols, 1,2-decanediol (INCI Decylene Glycol), diazolidinylurea, dichlorobenzyl alcohol, dimethyloxazolidine, DMDM hydantoin, dimethylhydroxmethylpyrazole, dehydroacetic acid, diazolidinylurea, DEDM hydantoin, DEDM hydantoin dilaurate, dibromopropamidine diisothionate, dimethylolethylenethiourea, dithio-methylbenzamide, DMHF, domiphen bromide, 7-ethylbicyclooxazolidine, ethylparaben, ethylhexylglycerol, ethanol, ethyl ferulate, formaldehyde, ferulic acid, glyceryl caprate, glutaral, glycerol formate, glyoxal, hexamidine diisethionate, hexanediol, hexetidine, hexamidine, hexamidinediparaben, hexamidineparaben, 4-hydroxybenzoic acid, hydroxymethyldioxazabicyclo-octane, imidazolidinylurea, imidiazolidinylurea NF, isobutylparaben, isothia-zolinone, iodopropynylbutyl carbamate, isodecylparaben, isopropylcresol, isopropylparaben, isopropyl sorbate, potassium sorbate NF FCC, copper usnate, potassium benzoate, potassium ethylparaben, potassium methylparaben, potassium paraben, potassium phenoxide, potassium o-phenylphenate, potassium propionate, potassium propylparaben, potassium salicylate, potassium sorbate, methylparaben, methylisothiazolinone, methylbenzethonium chloride phenol, methyldibromoglutaronitrile, methen-ammonium chloride, methylbromoglutaronitrile, magnesium benzoate, magnesium propionate, magnesium salicylate, MDM hydantoin, MEA benzoate, MEA o-phenylphenate, MEA salicylate, methylchloristhiazolin-one, sodium benzoate NF FCC, sodium caprylate, sodium dehydroacetate, sodium dehydroacetates FCC, sodium hydroxymethylglycinate, sodium methylparaben, sodium propylparaben, sodium iodoate, neem tree seed oil, nisin, sodium benzoate, sodium butylparaben, sodium p-chloro-m-cresol, sodium ethylparaben, sodium formate, sodium hydroxymethanesulfonate, sodium isobutylparaben, sodium paraben, sodium phenolsulfonate, sodium phenoxide, sodium o-phenylphenate, sodium propionate, sodium propylparaben, sodium pyrithione, sodium salicylate, sodium sorbate, orthol-phenylphenol, phenoxyethanol, propylparaben, polymethoxybicyclicoxa-zolidine, Pinus Pinaster bark extract, poloxamer 188, PVP iodine, para-bens, pircotone olamines, phenethyl alcohol, polyaminopropylbiguanide, polyquarternium-42, PEG-5 DEDM hydantoin, PEG-15 DEDM hydantoin, PEG-5 hydantoin oleate, PEG-15 DEDM hydantoin stearate, phenethyl alcohol, phenol, phenoxyethylparaben, phenoxyisopropanol, phenyl benzo-ate, phenyl mercury acetate, phenyl mercury benzoate, phenyl mercury borate, phenyl mercury bromide, phenyl mercury chloride, phenylparaben, o-phenylphenol, polyaminopropylbiguanide stearate, propionic acid, propyl benzoate, quaternium-15, quaternium-8, quaternium-14, Rosmarinus offici-nalis leaf extract, sorbic acid NF FCC, selenium disulfine, sorbic acid, salicylic acid, silver borosilicate, silver magnesium aluminium phosphate, triclosan, di-alpha-tocopherol, tocopherol acetate, thimersal, triclocarban, TEA sorbate, thimerosal, usnic acid, undecylenoyl PEG-5 paraben, Vitis vinifera seed extract, tea tree oil, hydrogen peroxide, zinc pyrithione, zinc oxide, zinc phenolsulfonate or combinations thereof.

Within this regard, the formulation may comprise the antimicrobially active compounds described in WO 2013/091775 A2, WO 2013/159865 A1 or WO 2013/167220 A1, in particular 4-hydroxy-cyclohexanecarboxylic acid butyl ester (available as RonaCare^{®} SereneShield from Merck KGaA, Darmstadt, Germany).

The formulation may further comprise anti-acne active compounds, for example, in WO2009/098139 on page 47, line 2 to page 48, line 27 and DE10324567, are conceivable. Illustrative further anti-acne active compounds are silver particles and silver salts, such as silver lactate and silver citrate, azelaic acid, ellagic acid, lactic acid, glycolic acid, salicylic acid, glycyrrhizinic acid, triclosan, phenoxyethanol, hexamidine isethionate, ketoconazole, peroxides, such as hydrogen peroxide or benzoyl peroxide, 3-hydroxybenzoic acid, 4-hydroxybenzoic acid, phytic acid, arachidonic acid, caprylyl glycol, ethylhexylglycerol, farnesol, cetylpyridinium salts, 6-trimethylpentyl-2-pyridone (Piroctone Olamine) and lipohydroxy acid (LHA).

Antidandruff active compounds are, for example, zinc pyrithione, Piroctone Olamine, selenium disulfide, Climbazole, Triclosan, Butylparaben, 1,3-Bis(hydroxymethyl)-5,5-dimethylimidazolidine-2,4-dione (DMDM Hydantoin), fumaric acid, Methylchloroisothiazolinone or Methylisothiazolinone (MIT).

The following, for example, may be mentioned as use form of the preparations: solutions, suspensions, emulsions, PIT emulsions, pastes, ointments, gels, creams, lotions, foams, masks, powders, soaps, surfactant-containing cleansing preparations, oils, aerosols, plasters, compresses, bandages and sprays, in particular for external application. Further application forms are, for example, sticks, shampoos and shower baths. Typcal cosmetic use forms are furthermore also lipsticks, lip-care sticks, powder, emulsion and wax make-up, and sun-protection, pre-sun and after-sun preparations.

Cosmetic and dermatological preparations can be, in particular, a water-free preparation, a lotion or emulsion, such as cream or milk, or microemulsion, in each case of the water-in-oil (W/O) type or of the oil-in-water (O/W) type, a multiple emulsion, for example of the water-in-oil-in-water (W/O/W) type or vice versa (O/W/O), gels or solutions (in particular oily-alcoholic, oily-aqueous or aqueous-alcoholic gels or solutions), a solid stick, an ointment or an aerosol. For application, the cosmetic and dermatological preparations according to the invention are applied to the skin in adequate amount in the usual manner for cosmetics.

Any desired conventional vehicles, assistants and, if desired, further active compounds may be added to the preparation. Preferred assistants originate from the group of the preservatives, stabilisers, solubilisers, colorants, i.e. pigments, dyes, emulsifiers or odour improvers.

Ointments, pastes, creams and gels may comprise the customary vehicles which are suitable for topical application, such as, for example, animal and vegetable fats, waxes, paraffins, starch, tragacanth, cellulose derivatives, polyethylene glycols, silicones, bentonites, silica, talc and titanium dioxide, or mixtures of these substances.

Powders and sprays may comprise the customary vehicles, such as, for example, lactose, talc, silica, aluminium hydroxide, calcium silicate and polyamide powder, or mixtures of these substances. Sprays may additionally comprise the customary readily volatile, liquefied propellants, such as, for example, chlorofluorocarbons, propane/butane or dimethyl ether. Compressed air can also advantageously be used. However, air can also be employed in pressureless metering devices, such as, for example, pump sprays.

Solutions and emulsions may comprise the customary vehicles, such as solvents, solubilisers and emulsifiers, such as, for example, water, ethanol, isopropanol, ethyl carbonate, ethyl acetate, benzyl alcohol, benzyl benzoate, propylene glycol, 1,3-butyl glycol, oils, in particular cottonseed oil, peanut oil, wheatgerm oil, olive oil, castor oil and sesame oil, glycerol fatty acid esters, polyethylene glycols and fatty acid esters of sorbitan, or mixtures of these substances.

A preferred solubiliser in general is 2-isopropyl-5-methylcyclohexane-carbonyl-D-alanine methyl ester.

Suspensions may comprise the customary vehicles, such as liquid diluents, such as, for example, water, ethanol or propylene glycol, suspension media, such as, for example, ethoxylated isostearyl alcohols, polyoxyethylene sorbitol esters and polyoxyethylene sorbitan esters, microcrystalline cellulose, aluminium metahydroxide, bentonite, agar-agar and tragacanth, or mixtures of these substances.

Soaps may comprise the customary vehicles, such as alkali-metal salts of fatty acids, salts of fatty acid monoesters, fatty acid protein hydrolysates, isothionates, lanolin, fatty alcohol, vegetable oils, plant extracts, glycerol, sugars, or mixtures of these substances.

Surfactant-containing cleansing products may comprise the customary vehicles, such as salts of fatty alcohol sulfates, fatty alcohol ether sulfates, sulfosuccinic acid monoesters, fatty acid protein hydrolysates, isothionates, imidazolinium derivatives, methyl taurates, sarcosinates, fatty acid amide ether sulfates, alkylamidobetaines, fatty alcohols, fatty acid glycerides, fatty acid diethanolamides, vegetable and synthetic oils, lanolin derivatives, ethoxylated glycerol fatty acid esters, or mixtures of these substances. Face and body oils may comprise the customary vehicles, such as synthetic oils, such as fatty acid esters, fatty alcohols, silicone oils, natural oils, such as vegetable oils and oily plant extracts, paraffin oils, lanolin oils, or mixtures of these substances.

Further suitable vehicles are liposomes, cyclodextrines or other sugars such as sorbitol.

A preferred embodiment is an emulsion which is in the form of a cream or milk and comprises, for example, the said fats, oils, waxes and other fatty substances, as well as water or an aqueous phase, for example with solvents or hydrophilic surfactants, and an emulsifier, as usually used for a preparation of this type.

The lipid phase can advantageously be selected from the following substance group:
- mineral oils, mineral waxes;
- oils, such as, for example, triglycerides of capric or caprylic acid, furthermore natural oils, such as, for example, castor oil;
- fats, waxes and other natural and synthetic fatty substances, preferably esters of fatty acids with alcohols having a low carbon number, for example with isopropanol, propylene glycol or glycerol, or esters of fatty alcohols with alkanoic acids having a low carbon number or with fatty acids;
- silicone oils, such as dimethylpolysiloxanes, diethylpolysiloxanes, diphenylpolysiloxanes, and mixed forms thereof.

The oil phase of the emulsions, oleogels or hydrodispersions or lipodispersions is advantageously selected from the group of esters of saturated and/or unsaturated, branched and/or unbranched alkanecarboxylic acids having a chain length of 3 to 30 C atoms and saturated and/or unsaturated, branched and/or unbranched alcohols having a chain length of 3 to 30 C atoms, from the group of the esters of aromatic carboxylic acid and saturated and/or unsaturated, branched and/or unbranched alcohols having a chain length of 3 to 30 C atoms. Ester oils of this type can then advantageously be selected from the group isopropyl myristate, isopropyl palmitate, isopropyl stearate, isopropyl oleate, n butyl stearate, n-hexyl laurate, n-decyl oleate, isooctyl stearate, isononyl stearate, isononyl isononanoate, 2-ethylhexyl palmitate, 2-ethylhexyl laurate, 2-hexaldecyl stearate, 2-octyldodecyl palmitate, oleyl oleate, oleyl erucate, erucyl oleate, erucyl erucate and synthetic, semi-synthetic and natural mixtures of esters of this type, such as, for example, jojoba oil. Furthermore, the oil phase can advantageously be selected from the group of branched and unbranched hydrocarbons and hydrocarbon waxes, silicone oils, dialkyl ethers, the group of saturated or unsaturated, branched or unbranched alcohols, and fatty acid triglycerides, specifically the triglycerol esters of saturated and/or unsaturated, branched and/or unbranched alkanecarboxylic acids having a chain length of 8 to 24, in particular 12-18 C atoms. The fatty acid triglycerides can advantageously be selected, for example, from the group of synthetic, semi-synthetic and natural oils, for example olive oil, sunflower oil, soya oil, peanut oil, rapeseed oil, almond oil, palm oil, coconut oil, palm kernel oil and the like.

Any desired mixtures of oil and wax components of this type may also advantageously be employed for the purposes of the present invention. It may also be advantageous to employ waxes, for example cetyl palmitate, as sole lipid component of the oil phase.

The aqueous phase of the preparations optionally advantageously comprises alcohols, diols or polyols having a low carbon number, and ethers thereof, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, furthermore alcohols having a low carbon number, such as, for example, ethanol, isopropanol, 1,2-propanediol, glycerol, and, in particular, one or more thickeners, which may advantageously be selected from the group of silicon dioxide, aluminium silicates, polysaccharides or derivatives thereof, such as, for example, hyaluronic acid, xanthan gum, hydroxypropylmethylcellulose, particularly advantageously from the group of the polyacrylates, preferably a polyacrylate from the group of the so-called Carbopols, for example Carbopol grades 980, 981, 1382, 2984, 5984, in each case individually or in combination. In particular, mixtures of the above-mentioned solvents are used. In the case of alcoholic solvents, water may be a further constituent.

In a preferred embodiment, the preparations comprise hydrophilic surfactants. The hydrophilic surfactants are preferably selected from the group of the alkylglucosides, acyl lactylates, betaines and coconut amphoacetates.

Emulsifiers that can be used are, for example, the known W/O and O/W emulsifiers. It is advantageous to use further conventional co-emulsifiers in the preferred O/W emulsions according to the invention.

The co-emulsifiers selected are advantageously, for example, O/W emulsifiers, principally from the group of substances having HLB values of 11-16, very particularly advantageously having HLB values of 14.5-15.5, so long as the O/W emulsifiers have saturated radicals R and R'. If the O/W emulsifiers have unsaturated radicals R and/or R', or if isoalkyl derivatives are present, the preferred HLB value of such emulsifiers may also be lower or higher.

It is advantageous to select the fatty alcohol ethoxylates from the group of the ethoxylated stearyl alcohols, cetyl alcohols, cetylstearyl alcohols (cetearyl alcohols).

It is furthermore advantageous to select the fatty acid ethoxylates from the following group:
polyethylene glycol (20) stearate, polyethylene glycol (21) stearate, polyethylene glycol (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate, polyethylene glycol (15) oleate, polyethylene glycol (16) oleate, polyethylene glycol (17) oleate, polyethylene glycol (18) oleate, polyethylene glycol (19) oleate, polyethylene glycol (20) oleate.

An ethoxylated alkyl ether carboxylic acid or salt thereof which can advantageously be used is sodium laureth-11 carboxylate. An alkyl ether sulfate which can advantageously be used is sodium laureth1-4 sulfate. An ethoxylated cholesterol derivative which can advantageously be used is polyethylene glycol (30) cholesteryl ether. Polyethylene glycol (25) soya-sterol has also proven successful. Ethoxylated triglycerides which can advantageously be used are the polyethylene glycol (60) evening primrose glycerides.

It is furthermore advantageous to select the polyethylene glycol glycerol fatty acid esters from the group of polyethylene glycol (20) glyceryl laurate, polyethylene glycol (21) glyceryl laurate, polyethylene glycol (22) glyceryl laurate, polyethylene glycol (23) glyceryl laurate, polyethylene glycol (6) glyceryl caprate/caprinate, polyethylene glycol (20) glyceryl oleate, polyethylene glycol (20) glyceryl isostearate, polyethylene glycol (18) glyceryl oleate (cocoate).

It is likewise favourable to select the sorbitan esters from the group polyethylene glycol (20) sorbitan monolaurate, polyethylene glycol (20) sorbitan monostearate, polyethylene glycol (20) sorbitan monoisostearate, polyethylene glycol (20) sorbitan monopalmitate and polyethylene glycol (20) sorbitan monooleate.

The following can be employed as optional W/O emulsifiers, but ones which may nevertheless be advantageous in accordance with the invention: fatty alcohols having 8 to 30 carbon atoms, monoglycerol esters of saturated and/or unsaturated, branched and/or unbranched alkanecarboxylic acids having a chain length of 8 to 24, in particular 12 to 18 C atoms, diglycerol esters of saturated and/or unsaturated, branched and/or unbranched alkanecarboxylic acids having a chain length of 8 to 24, in particular 12 to 18 C atoms, monoglycerol ethers of saturated and/or unsaturated, branched and/or unbranched alcohols having a chain length of 8 to 24, in particular 12 to 18 C atoms, diglycerol ethers of saturated and/or unsaturated, branched and/or unbranched alcohols having a chain length of 8 to 24, in particular 12 to 18 C atoms, propylene glycol esters of saturated and/or unsaturated, branched and/or unbranched alkanecarboxylic acids having a chain length of 8 to 24, in particular 12 to 18 C atoms, and sorbitan esters of saturated and/or unsaturated, branched and/or unbranched alkanecarboxylic acids having a chain length of 8 to 24, in particular 12 to 18 C atoms.

Particularly advantageous W/O emulsifiers are glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprinate, glyceryl monocaprylate or PEG-30 dipolyhydroxystearate.

The preparation may comprise cosmetic adjuvants which are usually used in this type of preparation, such as, for example, thickeners, softeners, moisturisers, surface-active agents, emulsifiers, preservatives, antifoams, perfumes, waxes, lanolin, propellants, dyes and/or pigments, which colour the composition itself or the skin, and other ingredients usually used in cosmetics.

The dispersant or solubiliser used can be an oil, wax or other fatty substances, a lower monoalcohol or a lower polyol or mixtures thereof. Particularly preferred monoalcohols or polyols include ethanol, i-propanol, propylene glycol, glycerol and sorbitol.

Further preferred embodiments are oily lotions based on natural or synthetic oils and waxes, lanolin, fatty acid esters, in particular triglycerides of fatty acids, or oily-alcoholic lotions based on a lower alcohol, such as ethanol, or a glycerol, such as propylene glycol, and/or a polyol, such as glycerol, and oils, waxes and fatty acid esters, such as triglycerides of fatty acids.

The preparation may also be in the form of an alcoholic gel which comprises one or more lower alcohols or polyols, such as ethanol, propylene glycol or glycerol, and a thickener, such as siliceous earth. The oily-alcoholic gels additionally comprise natural or synthetic oil or wax.

The solid sticks preferably consist of natural or synthetic waxes and oils, fatty alcohols, fatty acids, fatty acid esters, lanolin and other fatty substances.

If a preparation is formulated as an aerosol, the usual propellants, such as alkanes, air, nitrogen, dinitrogen monoxide, particularly preferably alkanes or air, are preferably used.

The present disclosure also relates to a formulation as described above, characterized in that a vehicle which is suitable for topical applications and optionally physiologically acceptable assistants and/or fillers are present. Such vehicle, assistants and fillers are defined as described above.

The present disclosure also relates to a process for the preparation of a preparation as described above, characterized in that the at least one compatible solute is mixed with the at least one further skin pore lightening active and optionally with the further ingredients.

These compounds can be incorporated into cosmetic or dermatological preparations in the usual manner.

The process for the preparation typically comprises the following steps:
(a) mixing of at least one compatible solute with at least one further pore-lightening active compound and at least one vehicle which is suitable for topical applications and optionally with physiologically acceptable assistants and/or fillers, and optionally (b) apparent making-ready of the compatible solutes. The preparations according to the invention can be prepared with the aid of techniques which are well known to the person skilled in the art. The mixing can result in dissolution, emulsification or dispersal of the at least one compatible solute, as described above, in the vehicle.

It goes without saying that the apparent making-ready is directed for the use for preventing and/or reducing cornification disorders in the skin or keratinized appendages to the skin. The apparent or appropriate making-ready can consist, for example, in: a) particular arrangement of the substance or matter, i.e. if these are individualised in such a way that suitability for the use in accordance with the patent is clearly evident; b) enclosure of use instructions (for example pack leaflet) on sale; c) formulation, assembly, dispensing and ready-to-use packaging; d) therapy plan, dose recommendation; e) use of a use-specific trade name (Schulte/Kühnen, German Patents Act, 8th Edition, § 14 marginal note 101).

The compositions as described above are applied to the skin or the keratinized appendages to the skin, such as the hair or nails.

The following examples are intended to illustrate the present invention.

### Examples

### Example 1: Proteomics approach

### Irradiation of samples and 2D-gelelectrophoresis:

### Materials and methods:

To assess and quantify protein oxidation (carbonylation) by differential in-gel electrophoresis proteomics approach evidencing differentially oxidized protein spots upon UV-A (365 nm) irradiation on skin explants in the presence or absence of ectoine (0,2%) (source Merck KGaA, Darmstadt, Germany).

During the study human explants are irradiated under UVA (LED source ; sources and chamber manufactured for custom needs by UWAVE;France, peak at 365nm at a 480J/cm₂ dose during 40 min). A solution of N-acetylcysteine (Reference A7250, Sigma-Aldrich) is used as positive control of protection. Just after irradiation, explants are snap-frozen and proteins extracted for analyses.

Protein extraction from explants is performed using a commercial kit (Total Protein Extraction Kit, Merck KGaA) protocol for skin explants. Extracted proteins are quantified by the Bradford method and split into equal amounts for analyses. The samples are analyzed for carbonylated proteins after they are labeled with fluorescent probes functionalized to selectively react with carbonyl groups. The fluorescent labeling of carbonyls is obtained by the reaction with amine-N-oxyl functionalized fluorophore (custom synthetized) and formation of a stable covalent bond.

Thus, the samples are labeled with two different fluorescent dyes characterized by different excitation and emission wavelength peaks: CF^{®}555 (Source :Biotium) is a red fluorescent dye spectrally similar to Cy3^{®} and Alexa Fluor^{®} 555 (λex547nm/λem572nm) and CF^{®}647 (Source: Biotium) CF^{®}647is a far-red fluorescent dye spectrally similar to Cy^{®}5 and Alexa Fluor^{®} 647 (λex639nm/λem668nm). This approach allows to load two distinct labeled samples in the same bi-dimensional electrophoresis gel and to analyze them independently.

Moreover, 50 µg of proteins of each sample are pooled and labeled a third fluorescent probe CF^{®}488 (Source: Biotium) (λex483nm/λem508nm). CF^{®}488 is a green fluorescent dye spectrally similar to Alexa Fluor^{®} 488. CF^{®}488 is and used as Internal Standard (IS) for inter-gel normalization. After specific fluorescent labeling, carbonylated proteins are separated by bi-dimensional electrophoresis and the fluorescence images are obtained for each specifically labeled sample.

The first dimension of protein separation (isoelectric focusing) is performed using gel-strips with nonlinear pH 3 to 11 while the second step of electrophoresis separation is conducted by SDS-PAGE using 4%-20% w/v gradient polyacrylamide gels. The digital acquisition of gel images is performed by using the «Ettan DIGE imager» system (GE Healthcare). Data treatment is performed with the software «Progenesis SameSpots» (Nonlinear Dynamics, UK).

Carbonylated proteins are presented as superposed images of two independent fluorescent signals (in green or red) and the coincident proteins with equivalent intensity result in yellow spots by superposition of the two dye colors used for their labeling (superposition of green and red colors). The carbonylated proteins that are present in a different level in the two samples on gel are visualized as red or green spots depending by the experimental group as independent labeling of each sample.

### Results:

More than 200 spots are detected per sample, with an increase in number and intensity in the samples of the irradiated group (UV-A). These spots are localized between 37 kDa and 75 kDa. The fluorescence signal of each spot is normalized over the internal standard spot signal for each gel. The alignment of spots from different gels and the analysis of variance of their carbonylation levels in different samples and in different groups are performed by using the software Progenesis Samespots.

The oxidative level of eight protein spots is detected as statistically significant increased in the irradiated (UV-A) group when compared to the control (not irradiated) group (p<0,05). In this group of 8 spots, five spots are detected as protected by the action of ectoine 0,2% (spots 186, 133, 184, 185, 206).

In the absence of irradiation ectoine also decreases the level of oxidation of ten protein spots that are different from the spots protected from UV-A stress (excepted for the spot 100).

These results indicate that ectoine protects distinct protein spots in basal conditions or upon irradiation, suggesting a potent detoxification action mediated by ectoine.

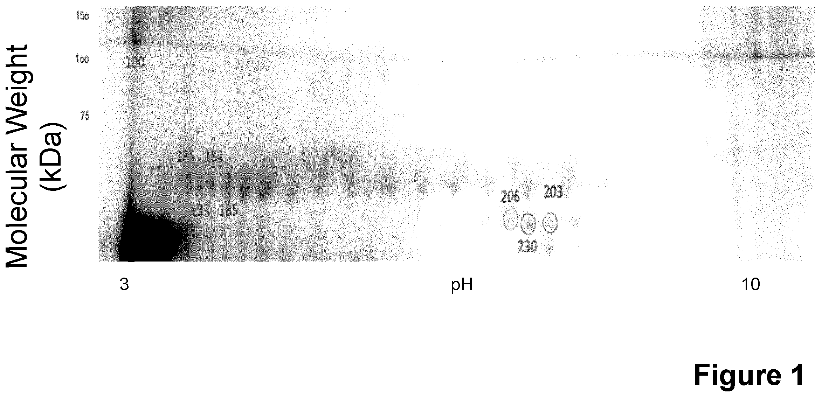

These results are shown in **Figure 1****:** 8 proteins are identified in 2D-Gelelectrophoresis to exhibit upregulated protein carbonylation by UV-A irradiation. 5 proteins exhibit reduced protein carbonylation by the treatment of 0.2% ectoine (spots 186, 133, 184, 185, 206). For 3 proteins (spots 100, 230, 203) carbonylation is not reduced after ectoine treatment. (n=4).

**Table 1** shows the resulting data: Data are processed from different stains:
CTRL: spot intensity without any treatment/irradiation
UV-A: spot intensity compared to CTRL after irradiation
UV-A + ectoine 0.2%: spot intensity with treatment of ectoine prior UV-A irradiation. (n=4)

**Table 1:**

| *Spot #* | *Average normalized volume (oxidation level)* | | | *Global statistics* | |
|---|---|---|---|---|---|
| | *CTRL (n=4)* | *UV-A (n=4)* | *UV-A + Ectoine 0.2%* | *Test Anova (p-value)* | *Overall fold change* |
| **185** | **0.934** | ↑ **1.543** | ↓ **0.994** | **0.002** | **1.7** |
| **184** | **0.916** | ↑ **1.639** | ↓ **1.026** | **0.006** | **1.8** |
| **133** | **0.968** | ↑ **1.675** | ↓ **0.988** | **0.029** | **1.7** |
| **186** | **0.837** | ↑ **1.354** | ↓ **0.952** | **0.038** | **1.6** |
| 100 | 0.778 | ↑ 1.339 | → 1.129 | 0.048 | 1.7 |
| **206** | **0.686** | ↑ **1.254** | ↓ **0.649** | **0.049** | **1.9** |
| 203 | 0.533 | ↑ 1.365 | → 1.062 | 0.052 | 2.6 |
| 230 | 0.576 | ↑ 1.229 | → 1.433 | 0.075 | 2.5 |

### Identification of proteins:

### Materials and methods:

Bi-dimensional electrophoresis gels are fixed and proteins stained using Coomassie blue (G-250, Reference 27815 (Sigma-Aldrich)). Selected spots are excised and digested with LysC/Trypsine (Mass Spec grade (Promega)) according to commercial digestion kit (Trypsin/Lys-C ;ix, Mass Spec gradePromega) after reduction and alkylation of gel fragments.

Peptide mass spectral signals are collected by using nanoLC RSLC/ESI QExactive (Thermo Scientific, Orbitrap).

The list of peptides peak intensities and mass-to-charge (m/z) values is analyzed with Mascot (matrice Science Ltd, London, UK) and SEQUEST HT (Thermo Scientific, 2013) according to the following parameters:
**Software Mascot v2.5** and **SEQUEST HT,** via **Proteome Discoverer** v2.2
**Type of search** MS/MS Ion Search
**Database** SwissProt 03/2018 (554,241 sequences; 198,410,167 residues)
**Taxonomy** Homo sapiens (human)
**Enzyme** Trypsin
**Variable Modifications**
**Digestion - Desalting**
Carbamidomethyl (C), Deamidated (NQ), Oxidation (M)
LysC/Trypsine - Zip-tip, Elution with 50% ACN/0.1%TFA
**Liquid Chromatography** 60 min gradient 2-40% phase B (phase A 98% H₂O 0.1% formic acid 2% ACN, phase B 90% ACN, 10% H₂O 0.1% formic acid), 6µL of injection, column Acclaim PepMap100 C18, 50 cm, 75 µm **Mass Values** Monoisotopic
**Protein mass** Unrestricted
**Peptide Mass Tolerance** 5 ppm
**Fragment Mass Tolerance** 0.5 Da
**Peptide Charge** 2+ et 3+
**Max Missed Cleavages** 2
**Instrument Type** RSLC/ESI QExactive (Orbitrap)
**Significance threshold** p<0.05
**MS/MS** DDA top 10, resolution MS 70 000, resolution MSMS 17 500 The UniProt database (03/2018) restricted on « human » taxon is interrogated by the software Protein Discoverer 2.2 using the search engines Mascot et SEQUEST.

### Results:

The results are shown in **Table 2:**

**Table 2:**

| *Spot #* | *Protein* | | *%Cov₃* | *Unique identified peptides⁴* | *Score sequest HT* |
|---|---|---|---|---|---|
| | *Identified¹* | *Access number²* | | | |
| 185 | Filaggrin-2 | Q5D862 | 1 | 2 | 8.07 |
| 184 | Hornerin | Q86YZ3 | 8 | 6 | 20.26 |
| 133 | Hornerin | Q86YZ3 | 6 | 5 | 16.51 |
| 186 | Keratin, type II cytoskeletal 1 | P04264 | 58 | 46 | 412.29 |
| 206 | Catalase | P04040 | 15 | 6 | 23.8 |

| | | | | | |
|---|---|---|---|---|---|
| 1 : protein name (SwissProt or NCBInr) 2 : accession code in SwissProt database (http://expasy.org/) 3 : sequence coverage (percentage) 4 : number of unique identified peptides 5 : Score Sequest HT : score of the Sequest HT search algorithm. Proteins are identified over a threshold value resulting from the protein sequence and the identified peptides (Xcorr, Thermo Scientific, 2013). | | | | | |

In this example it is shown in the differential protein analysis that ectoine prevents protein carbonylation significantly in proteins with known functionality in the skin barrier function of the stratum corneum.

### Example 2: Fluorescence imaging

To evidence changes at the expression level of a selected pool of proteins on skin explants (*ex vivo*), treated or not with ectoine 0.2% (source Merck KGaA. Darmstadt, Germany) and exposed or not to UV-A irradiation: At day 0 (D0), the skin explants from a female donor (34 years old, phototype III, ref. EXP001100F022) are kept alive in calcium-free DMEM (Dulbecco's modified Eagle medium), with 10% FCS (Fetal calf serum), at 37°C and humid atmosphere, supplemented with 5% CO₂. On day 1 (D1), ectoine 0.2% w/v) is obtained by dilution in H₂O. The experimental groups are incubated for 24 hours with the active prior to irradiation.

On day 2 (D2), a stress induced by irradiation with UV-A (LED source, peak at 365 nm at a 480J/cm² during 40 minutes) is applied to the explants.

### Materials and methods:

Skin explants are previously treated or not with ectoine and irradiated or not with UV-A, cryo-preserved in OCT (an embedding resin (Shandon Cryomatrix; Thermo Scientific, Reference 6769006)), flash-frozen in isopetan/liquid nitrogen and stored at -80 °C.

Tissue sections of 5 µm are cut by using a cryotome, air-dried at room temperature (RT) for 15 minutes and fixed with a mix of 95% ethanol/5% acetic acid for 5 minutes. Fixed tissues are then saturated with 5% BSA (bovine serum albumin) in PBS (Phosphate Buffered Saline; 1X, pH 7.4) at RT.

Primary antibodies (Filaggrin 2, Polyclonal, Invitrogen^{™} Rabbit Polyclonal Antibody, Ref: Thermo Scientific PA555951 Dilution 1/100; LAMP-2A, Polyclonal, Invitrogen^{™}Rabbit Polyclonal Antibody Ref: Invitrogen 512200 Dilution 1/200; Filaggrin, santa cruz biotechnology Mouse Antibody SC-66192 Dilution 1/50) are diluted in PBS/5% BSA and applied on tissues for 1h at RT followed by 3 steps of wash with PBS pH 7.4 of 5 minutes each.

The appropriate secondary antibody diluted in PBS (Goat anti-Mouse IgG (H+L) Cross-Adsorbed, Alexa Fluor 488, Polyclonal, Secondary Antibody, Invitrogen^{™} Goat Polyclonal Secondary Antibody Ref: Invitrogen R37120 Dillution :1/1000; Goat anti-Rabbit IgG (H+L) Cross-Adsorbed, Alexa Fluor 488, Polyclonal, Secondary Antibody, Invitrogen^{™} Goat Polyclonal Secondary Antibody Ref: Invitrogen A11008 ) and DAPI (dilution 1:3000) are applied on tissue sections for 1h at RT followed by 3 steps of wash with PBS pH 7.4 of 5 minutes each.

Fluorescent images are collected with an epi-fluorescent microscope (DMi8, Leica) and treated with ImageJ software (Schneider, C.A., Rasband, W.S., Eliceiri, K.W. "NIH Image to ImageJ: 25 years of image analysis". Nature Methods 9, 671-675, 2012). Image comparisons between different conditions are achieved using identical conditions of acquisition (100ms and 630X).

### Results:

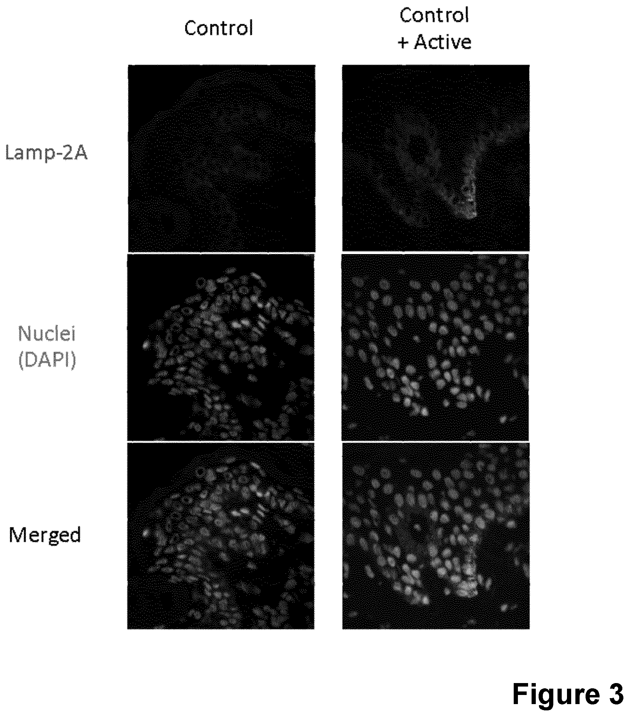

The results are shown in Figures 2 and 3:
Figure 2 shows that Filaggrin expression level decreases upon UV-A irradiation compared to the non-irradiated explants. However the ectoine ("Active") treatment prevents this decrease of Filaggrin upon UV-A irradiation.
Figure 3 shows that ectoine ("Active") treatment in basal conditions (without UV-A) increases LAMP2A expression contributing to the activity of chaperone -mediated autophagy (CMA) detoxification function of the skin.

### Example 3: O/W Day-Care Formulation

| **Ingredients** | **Art. No.** | | **INCI (CTFA)** | **[%]** |
|---|---|---|---|---|
| **A** | | | | |
| Water, demineralized | | | AQUA (WATER) | ad 100 |
| **RonaCare^{®} Ectoin** | **1.30200** | **(1)** | **ECTOINE** | **1.00** |
| Glycerol anhydrous (vegetable) EMPROVE^{®} bio | 1.37028 | (1) | GLYCERIN | 4.25 |
| Solagum AX | | (2) | XANTHAN GUM, ACACIA GUM | 0.70 |

| **B** | | | | |
|---|---|---|---|---|
| Montanov^{™} L | | (2) | C14-22 ALCOHOLS, C12-20 ALKYL GLUCOSIDE | 4.00 |
| Montanov^{™} 14 | | (2) | MYRISTYL ALCOHOL, MYRISTYL GLUCOSIDE | 2.00 |
| LIPOCIRE A SG | | (3) | C10-18 TRIGLYCERIDES | 3.00 |
| Cetiol^{®} SB 45 | | (4) | BUTYROSPERMUM PARKII BUTTER (BUTYROSPERMUM PARKII (SHEA) BUTTER) | 5.00 |
| Mango butter XNE003 | | (5) | MANGIFERA INDICA SEED BUTTER (MANGIFERA INDICA (MANGO) SEED BUTTER) | 5.00 |
| Crodamol ISIS-LQ-(MV) | | (6) | ISOSTEARYL ISOSTEARATE | 5.00 |
| Compritol 888 CG PELLETS | | (3) | GLYCERYL BEHENATE | 3.00 |

| **C** | | | | |
|---|---|---|---|---|
| Water, demineralized | | | AQUA (WATER) | 5.00 |
| RonaCare^{®} Potassium Sorbate | 1.32144 | (1) | POTASSIUM SORBATE | 0.60 |
| RonaCare^{®} Benzyl Alcohol | 1.30153 | (1) | BENZYL ALCOHOL | 1.00 |

| **D** | | | | |
|---|---|---|---|---|
| Citric Acid 30% | 100242 | (1) | AQUA (WATER), CITRIC ACID | q.s. |

### Procedure:

Disperse Solagum AX in the water and stir until homogeneous. Add ectoine and glycerin and stir until homogeneous.

Heat phases A and B separately to 80°C. Stir phase B into phase A. Homogenize. Cool down while stirring and add the preservatives (dissolve potassium sorbate in water). Adjust pH value under 6.00 with citric acid.

### Suppliers:

| | | |
|---|---|---|
| (1) Merck KGaA, Darmstadt, Germany/EMD Performance Materials | (2) | Seppic |
| (3) Gattefossé (Deutschland) GmbH | (4) | BASF AG |
| (5) Greentech SA | (6) | Croda |

### Example 4: W/O Night care formulation

| **Ingredients** | **Art. No.** | | **INCI (CTFA)** | **[%]** |
|---|---|---|---|---|
| **A** | | | | |
| **RonaCare^{®} Ectoin** | **1.30200** | **(1)** | **ECTOINE** | **1.00** |
| Glycerol 85% | 1.04091 | (1) | GLYCERIN, AQUA (WATER) | 2.00 |
| RonaCare^{®} Magnesium Sulfate | 1.32219 | (1) | MAGNESIUM SULFATE | 1.20 |
| Water, demineralized | | | AQUA (WATER) | ad 100 |

| **B** | | | | |
|---|---|---|---|---|
| Dermofeel^{®} PGPR | | (2) | POLYGLYCERYL-3 POLYRICINOLEATE | 3.00 |
| Lameform TGI | | (3) | POLYGLYCERYL-3 DIISOSTEARATE | 2.00 |
| Beeswax yellow | | (4) | CERA ALBA (BEESWAX) | 0.50 |
| Tegosoft^{®} DC | | (5) | DECYL COCOATE | 8.00 |
| Cetiol^{®} J 600 | | (3) | OLEYL ERUCATE | 7.00 |
| Miglyol^{®} 818 | | (6) | CAP RYLIC/CAPRIC/LINOLEIC TRIGLYCERIDE | 5.00 |
| Ceraphyl 791 | | (7) | ISOCETYL STEAROYL STEARATE | 2.00 |

| **C** | | | | |
|---|---|---|---|---|
| RonaCare^{®} RenouMer | 1.32277 | (1) | AQUA (WATER), POLYSIPHONIA ELONGATA EXTRACT (ALGAE EXTRACT), SODIUM BENZOATE, CITRIC ACID | 2.00 |
| Preservatives | | | | q.s. |
| Fragrance | | | PARFUM | q.s. |

### Procedure:

Heat phases A and B to 75°C. Stir phase A into phase B. Homogenize.

Cool down while stirring. Add ingredients of phase C step by step.

### Suppliers:

| | | |
|---|---|---|
| (1) Merck KGaA, Darmstadt, Germany/EMD Performance Materials | (2) | Dr. Straetmans |
| (3) BASF AG | (4) | Gustav Heess GmbH |
| (5) Evonik Nutrition & Care GmbH | (6) | IOI Oleo GmbH |
| (7) ISP Global Technologies | | |

### Example 5: O/W Formulation baby care lotion

| **Ingredients** | **Art. No.** | | **INCI (CTFA)** | **[%]** |
|---|---|---|---|---|
| **A** | | | | |
| **RonaCare^{®} Ectoin** | **1.30200** | **(1)** | **ECTOINE** | **1.00** |
| Glycerol 85% | 1.04091 | (1) | GLYCERIN, AQUA (WATER) | 10.00 |
| Keltrol^{®} CG-SFT | | (2) | XANTHAN GUM | 0.30 |
| Avicel RC-591F | | (3) | MICROCRYSTALLINE CELLULOSE, CELLULOSE GUM | 2.00 |
| Montanov^{™} L | | (4) | C14-22 ALCOHOLS, C12-20 ALKYL GLUCOSIDE | 3.00 |
| Montanov^{™} 14 | | (4) | MYRISTYL ALCOHOL, MYRISTYL GLUCOSIDE | 2.00 |
| Water, demineralized | | | AQUA (WATER) | 57.58 |

| **B** | | | | |
|---|---|---|---|---|
| Oxynex^{®} L-CV Liquid | 1.32330 | (1) | ALCOHOL, TOCOPHEROL, CAPRYLIC/CAPRIC TRIGLYCERIDE, ASCORBYL PALMITATE, ASCORBIC ACID, CITRIC ACID | 0.10 |
| Myritol^{®} 318 | | (5) | CAPRYLIC/CAPRIC TRIGLYCERIDE | 10.00 |
| Cetiol^{®} SB 45 | | (5) | BUTYROSPERMUM PARKII BUTTER (BUTYROSPERMUM PARKII (SHEA) BUTTER) | 10.00 |

| **C** | | | | |
|---|---|---|---|---|
| RonaFlair^{®} Soft Sphere | 1.17756 | (1) | SYNTHETIC FLUORPHLOGOPITE, SILICA | 2.00 |

| **D** | | | | |
|---|---|---|---|---|
| Microcare^{®} Emollient APHX | | (6) | PHENYLPROPANOL, 1,2-HEXANEDIOL | 1.00 |
| RonaCare^{®} Bisabolol nat. | 1.30170 | (1) | BISABOLOL | 1.00 |

| **E** | | | | |
|---|---|---|---|---|
| Sodium Hydroxide 20% | | | AQUA, SODIUM HYDROXIDE | 0.02 |

### Procedure:

Sprinkle Avicel RC-591F in water and part of the glycerin (7%) and stir for about 20 min. Add premixed xanthan gum and remaining glycerin (3%) and stir until homogeneous. Solubilize RonaCare^{®} Ectoin while stirring. Then add Montanov^{™} L and Montanov^{™} 14. Prepare phase B and heat phase A and phase B to 75-78°C. Add phase B into phase A and homogenize. Add phase C and stir until homogeneous. Cool down slowly while gently stirring.

At T<35°C, add phase D and stir until homogeneous.

Adjust pH between 6.8-7 with phase E.

### Suppliers:

| | | | |
|---|---|---|---|
| (1) | Merck KGaA, Darmstadt, Germany/EMD Performance Materials | (2) | Azelis Germany GmbH |
| (3) | Soliance | (4) | Seppic |
| (5) | BASF AG | (6) | Thor Personal Care SAS |

### Example 6: Day Care Gel Formulation

| **Ingredients** | **Art. No.** | | **INCI (CTFA)** | **[%]** |
|---|---|---|---|---|
| **A** | | | | |
| Eusolex^{®} UV-Pearls^{®} OB-S X | 1.32704 | (1) | AQUA (WATER, AVOBENZONE), OCTOCRYLENE, SORBITOL, BUTYL METHOXYDIBENZOYLMETHANE, SILICA, PVP | 20.00 |
| Eusolex^{®} UV-Pearls^{®} 2292 | 1.30801 | (1) | AQUA (WATER), ETHYLHEXYL METHOXYCINNAMATE (OCTYL METHOXYCINNAMATE), SILICA, PVP, PHENOXYETHANOL, CHLORPHENESIN, DISODIUM EDTA | 8.00 |
| **RonaCare^{®} Ectoin** | **1.30200** | **(1)** | **ECTOINE** | 0.30 |
| Carbopol^{®} Ultrez 10 Water, demineralized | | (2) | CARBOMER AQUA (WATER) | 0.50 ad 100 |

| **B** | | | | |
|---|---|---|---|---|
| Sodium Hydroxide, 10% | 1.05588 | (1) | AQUA (WATER), SODIUM HYDROXIDE | 1.10 |

| **C** | | | | |
|---|---|---|---|---|
| RonaCare^{®} AP | 1.30163 | (1) | BIS-ETHYLHEXYL HYDROXYDIMETHOXY BENZYLMALONATE | 1.00 |
| RonaCare^{®} Cyclopeptide-5 alcoholfree | 1.32398 | (1) | GLYCERIN, AQUA (WATER), LECITHIN, ECTOINE, CYCLOTETRAPEPTIDE-24 AMINOCYCLOHEXANE CARBOXYLATE | 4.00 |
| Preservatives | | | | q.s. |
| Fragrance | | | PARFUM | q.s. |

### Procedure:

Disperse the Carbomer in water, then add rest of phase A. Adjust pH value to approx. 5.5 -6.0 with phase B. Add the

ingredients of phase C separately and mix until uniform.

### Suppliers:

| | | | | |
|---|---|---|---|---|
| (1) | Merck KGaA, Darmstadt, Germany/EMD Performance Materials | (2) | Gattefossé (Deutschland) GmbH | |

### Example 7: SunCare - Gel formulation

| **Ingredients** | **Art. No.** | | **INCI (CTFA)** | **[%]** |
|---|---|---|---|---|
| **A** | | | | |
| Eusolex^{®} 9020 | 1.05844 | (1) | BUTYL METHOXYDIBENZOYLMETHANE (AVOBENZONE) | 5.00 |
| Eusolex^{®} OCR | 1.05377 | (1) | OCTOCRYLENE | 5.00 |
| Eusolex^{®} S | 1.32276 | (1) | BIS-ETHYLHEXYLOXYPHENOL METHOXYPHENYL TRIAZINE (NOT REGISTERED) | 4.70 |
| Uvasorb^{®} HEB | | (2) | DIETHYLHEXYL BUTAMIDO TRIAZONE | 3.70 |
| Tego Care PBS 6 | | (3) | POLYGLYCERYL-6 STEARATE, POLYGLYCERYL-6 BEHENATE | 3.00 |
| Lanette^{®} 18 | | (4) | STEARYL ALCOHOL | 0.50 |
| Tegin M Pellets | | (3) | GLYCERYL STEARATE | 0.50 |
| Tegosoft^{®} XC | | (3) | PHENOXYETHYL CAPRYLATE | 3.00 |
| DUB VCI 10 | | (5) | ISODECYL NEOPENTANOATE | 4.00 |
| Keltrol^{®} CG-SFT | | (6) | XANTHAN GUM | 0.20 |

| **B** | | | | |
|---|---|---|---|---|
| Eusolex^{®} T-AVO | 1.05335 | (1) | TITANIUM DIOXIDE (NANO), SILICA | 3.00 |
| **RonaCare^{®} Ectoin** | **1.30200** | **(1)** | **ECTOINE** | **1.00** |
| Glycerol, anhydrous | 1.04093 | (1) | GLYCERIN | 2.00 |
| Avicel PC 591 | (5) | MICROCRYSTALLINE CELLULOSE, CELLULOSE GUM | 1.00 | |
| Water, demineralized | | AQUA (WATER) | ad 100 | |

| **C** | | | | |
|---|---|---|---|---|
| Preservatives | | | q.s. | |

### Procedure:

Disperse Avicel PC 591 in water and add than the remaining ingredients of phase B. Heat phases A and B to 80°C and disperse Keltrol into the oilphase. Stir phase A into phase B. Homogenize. Cool down while stirring.

Add phase C and adjust pH if necessary.

### Suppliers:

| | | | |
|---|---|---|---|
| (1) | Merck KGaA, Darmstadt, Germany/EMD Performance Materials | (2) | 3V Sigma |
| (3) | Evonik Nutrition & Care GmbH | (4) | BASF AG |
| (5) | Azelis Germany GmbH | (6) | RAHN GmbH |

### Example 8: W/O BB Formulation with sun protection

| **Ingredients** | **Art. No.** | | **INCI (CTFA)** | **[%]** |
|---|---|---|---|---|
| **A1** | | | | |
| Eusolex^{®} T-S | 1.05334 | (1) | TITANIUM DIOXIDE (NANO), ALUMINA, STEARIC ACID | 5.00 |
| RonaCare^{®} AP | 1.30163 | (1) | BIS-ETHYLHEXYL HYDROXYDIMETHOXY BENZYLMALONATE | 1.00 |
| RonaCare^{®} Bisabolol nat. | 1.30170 | (1) | BISABOLOL | 0.20 |
| Abil EM 90 | | (2) | CETYL PEG/PPG-10/1 DIMETHICONE | 2.00 |
| Isolan GO 33 | | (2) | POLYGLYCERYL-3 OLEATE | 1.00 |
| Beeswax Yellow | 1.11545 | (1) | CERA ALBA (BEESWAX) | 1.00 |
| Shea Butter | | (3) | BUTYROSPERMUM PARKII BUTTER (BUTYROSPERMUM PARKII (SHEA) BUTTER) | 1.00 |
| Isopropyl Palmitate | | (4) | ISOPROPYL PALMITATE | 5.00 |
| Tegosoft^{®} TN | | (2) | C12-15 ALKYL BENZOATE | 2.00 |
| Xiameter^{®} PMX-200 | | (5) | DIMETHICONE | 2.00 |
| Silicone Fluid (100cs) | | | | |
| Soybean Oil | | (6) | GLYCINE SOJA (GLYCINE SOJA (SOYBEAN) OIL) | 8.00 |
| Wheat Germ Oil | | (6) | TRITICUM VULGARE (TRITICUM VULGARE (WHEAT) GERM OIL) | 2.00 |
| Oxynex^{®} K liquid | 1.08324 | (1) | PEG-8, TOCOPHEROL, ASCORBYL PALMITATE, ASCORBIC ACID, CITRIC ACID | 0.10 |

| **A2** | | | | |
|---|---|---|---|---|
| Titan(IV)-oxid | 1.00805 | (1) | TITANIUM DIOXIDE | 8.99 |
| Unipure Yellow LC 182 | | (7) | CI 77492 (IRON OXIDES) | 0.75 |
| Unipure Red LC 381 | | (7) | CI 77491 (IRON OXIDES) | 0.15 |
| Unipure Brown LC 889 | | (7) | CI 77491 (IRON OXIDES), CI 77499 (IRON OXIDES) | 0.07 |
| Unipure Blue LC 686 | | (7) | CI 77007 (ULTRAMARINE BLUE) | 0.04 |

| **B** | | | | |
|---|---|---|---|---|
| **RonaCare^{®} Ectoin** | 1.30200 | (1) | **ECTOINE** | **0.50** |
| 1,2-Propanediol | 1.07478 | (1) | PROPYLENE GLYCOL | 4.00 |
| RonaCare^{®} Sodium Chloride Water, demineralized | 1.32260 | (1) | SODIUM CHLORIDE AQUA (WATER) | 0.50 ad 100 |

| C | | | | |
|---|---|---|---|---|
| Fragrance | | | PARFUM | q.s. |
| Preservatives | | | | q.s. |

### Procedure:

Heat phase A1 up to 70-80°C. Add phase A2 to phase A1 while stirring.

Heat phase A and phase B up to 70-80°C. Slowly add phase B to phase A under strong mixing. Cool down to room temperature.

### Suppliers:

| | | | |
|---|---|---|---|
| (1) | Merck KGaA, Darmstadt, Germany/EMD Performance Materials | (2) | Evonik Nutrition & Care GmbH |
| (3) | H. Erhard Wagner GmbH | (4) | BASF AG |
| (5) | Biesterfeld | (6) | Gustav Heess GmbH |
| (7) | S. Goldmann GmbH & Co. KG | | |

### Example 9: Solution/ 2Phase systems - Face Mist Formulation

| **Ingredients** | **Art. No.** | | **INCI (CTFA)** | **[%]** |
|---|---|---|---|---|
| **A** | | | | |
| RonaCare^{®} AP | 1.30163 | (1) | BIS-ETHYLHEXYL HYDROXYDIMETHOXY BENZYLMALONATE | 1.00 |
| RonaCare^{®} Pristine Bright^{™} | 1.32720 | (1) | METHOXYPHENYL T-BUTYLPHENYL PROPANEDIOL | 0.50 |
| Polyaldo 10-1-S | | (2) | POLYGLYCERYL-10 STEARATE | 4.00 |
| Dermofeel^{®} TC-7 | | (3) | TRIHEPTANOIN | 1.00 |
| DUB APRILOSE + | | (2) | APRICOT KERNEL OIL POLYGLYCERYL-4 ESTERS | 3.00 |
| PELEMOL^{®} DISM | | (4) | DIISOSTEARYL MALATE | 4.00 |

| **B** | | | | |
|---|---|---|---|---|
| RonaCare^{®} **Ectoin** | **1.30200** | **(1)** | **ECTOINE** | **0.50** |
| 1,2-Propanediol | 1.07478 | (1) | PROPYLENE GLYCOL | 3.00 |
| Rhodicare XC | | (2) | XANTHAN GUM | 0.20 |
| Avicel PC 611 | | (2) | MICROCRYSTALLINE CELLULOSE, CELLULOSE GUM | 1.00 |
| Water, demineralized | | | AQUA (WATER) | ad |
| | | | | 100 |

| **C** | | | | |
|---|---|---|---|---|
| Citric acid solution 10% | | | AQUA, CITRIC ACID | q.s. |
| RonaFlair^{®} Balance Gold | 1.17734 | (1) | CI 77891 (TITANIUM DIOXIDE), MICA, TIN OXIDE | 1.50 |
| RonaFlair^{®} Flawless | 1.17789 | (1) | SILICA, CI 77891 (TITANIUM DIOXIDE), CI 77491 (IRON OXIDES) | 3.00 |
| RonaCare^{®} ASC III^{®} | 1.10154 | (1) | AQUA (WATER), HYDROGENATED LECITHIN, DIPALMITOYL HYDROXYPROLINE, LECITHIN, PHENOXYETHANOL, MANNITOL, BETA-SITOSTEROL, LINOLEIC ACID, TOCOPHEROL, SODIUM ASCORBATE | 3.00 |
| Preservatives | | | | q.s. |

### Procedure:

Disperse PC Avicel 611 in water and stir at least 10 minutes. Add the remaining ingredients of phase B. Smartly heating for better dissolving of RonaCare^{®} Pristine Bright^{™} is possible. Prepare phase A. Add phase A to phase B while stirring. Homogenize. Adjust pH value to 5.5 to 6.5. Add the ingredients of phase C separately.

Suppliers:

| | |
|---|---|
| (1) Merck KGaA, Darmstadt, Germany/EMD Performance Materials | (2) Azelis Germany GmbH |
| (3) Dr. Straetmans | (4) Phoenix Chemical, Inc. |

### Example 10: Solution/2-phase systems - suncare 2-phase spray

| **Ingredients** | **Art. No.** | | **INCI (CTFA)** | **[%]** |
|---|---|---|---|---|
| **A** | | | | |
| Eusolex^{®} 232 | 1.05372 | (1) | PHENYLBENZIMIDAZOLE SULFONIC ACID | 4.00 |
| **RonaCare^{®} Ectoin** | **1.30200** | **(1)** | **ECTOINE** | **1.00** |
| RonaCare^{®} Sodium Chloride | 1.32260 | (1) | SODIUM CHLORIDE | 0.50 |
| Glycerol 85% | 1.04091 | (1) | GLYCERIN, AQUA (WATER) | 10.00 |
| Triethanolamine, EMPROVE^{®} exp. Water, demineralized | 1.08372 | (1) | TRIETHANOLAMINE | 2.30 |
| | | | AQUA (WATER) | ad |
| | | | | 100 |

| **B** | | | | |
|---|---|---|---|---|
| Eusolex^{®} OS | 1.06949 | (1) | ETHYLHEXYL SALICYLATE (OCTYL SALICYLATE) | 5.00 |
| RonaCare^{®} AP | 1.30163 | (1) | BIS-ETHYLHEXYL HYDROXYDIMETHOXY BENZYLMALONATE | 2.00 |
| Eusolex^{®} HMS | 1.11412 | (1) | HOMOSALATE | 10.00 |
| Eusolex^{®} OCR | 1.05377 | (1) | OCTOCRYLENE | 4.00 |
| Eusolex^{®} 9020 | 1.05844 | (1) | BUTYL METHOXYDIBENZOYLMETHANE (AVOBENZONE) | 5.00 |
| Tegosoft^{®} TN | | (2) | C12-15 ALKYL BENZOATE | 6.00 |
| Cetiol^{®} C5 | | (3) | COCO CAPRYLATE | 10.50 |
| Creasil IH CG | | (4) | ISOHEXADECANE | 10.50 |

| **C** | | | | |
|---|---|---|---|---|
| Preservatives | | | PARFUM | q.s. |
| Fragrance | | | | q.s. |

| **D** | | | | |
|---|---|---|---|---|
| Dragocolor Brilliantblue FCF 85, 1% in water | | (5) | AQUA (WATER), CI 42090 (FD&C BLUE NO. 1) | 0.11 |

### Procedure:

Heat phase B to 75°C and stir until a homogeneous phase is reached. Cool it down to room temperature.

Mix the ingredients of phase A together and stir until homogeneous. Adjust pH to 7,2 if necessary.

Add C to B and then add B+C to A and mix. Add phase D.

### Suppliers:

| | |
|---|---|
| (1) Merck KGaA, Darmstadt, Germany/EMD Performance Materials | (2) Evonik Nutrition & Care GmbH |
| (3) BASF AG | (4) The Innovation Company |
| (5) Cosnaderm GmbH | |

### Example 11: W/O Soft cream with organic/inorganic filters

| **Ingredients** | **Art. No.** | | **INCI (CTFA)** | **[%]** |
|---|---|---|---|---|
| **A1** | | | | |
| Eusolex^{®} OCR | 1.05377 | (1) | OCTOCRYLENE | 4.00 |
| Eusolex^{®} OS | 1.06949 | (1) | ETHYLHEXYL SALICYLATE (OCTYL SALICYLATE) | 4.00 |
| Eusolex^{®} 9020 | 1.05844 | (1) | BUTYL METHOXYDIBENZOYLMETHANE (AVOBENZONE) | 5.00 |
| Uvinul^{®} T-150 | | (2) | ETHYLHEXYL TRIAZONE (NOT REGISTERED) | 1.50 |
| Eusolex^{®} S | 1.32276 | (1) | BIS-ETHYLHEXYLOXYPHENOL METHOXYPHENYL TRIAZINE (NOT REGISTERED) | 1.80 |
| Uvasorb^{®} HEB | | (3) | DIETHYLHEXYL BUTAMIDO TRIAZONE | 0.80 |
| RonaCare^{®} AP | 1.30163 | (1) | BIS-ETHYLHEXYL HYDROXYDIMETHOXY BENZYLMALONATE | 1.00 |
| Amphisol^{®} K | | (4) | POTASSIUM CETYL PHOSPHATE | 1.80 |
| Tegin^{®} M Pellets | | (5) | GLYCERYL STEARATE | 1.20 |
| Lanette^{®} O | | (2) | CETEARYL ALCOHOL | 1.40 |
| Cetiol^{®} CC | | (2) | DICAPRYLYL CARBONATE | 3.00 |
| Tegosoft^{®} TN | | (5) | C12-15 ALKYL BENZOATE | 2.00 |
| Xiameter^{®} PMX-0245 | | (6) | CYCLOPENTASILOXANE | 4.00 |

| **A2** | | | | |
|---|---|---|---|---|
| Eusolex^{®} T-AVO | 1.05335 | (1) | TITANIUM DIOXIDE (NANO), SILICA | 1.50 |

| **B** | | | | |
|---|---|---|---|---|
| Eusolex^{®} 232 | 1.05372 | (1) | PHENYLBENZIMIDAZOLE SULFONIC ACID | 1.20 |
| **RonaCare^{®} Ectoin** | **1.30200** | **(1)** | **ECTOINE** | **0.50** |
| Glycerol 85% | 1.04091 | (1) | GLYCERIN, AQUA (WATER) | 5.00 |
| RonaCare^{®} Disodium EDTA | 1.32221 | (1) | DISODIUM EDTA | 0.05 |
| RonaCare^{®} Tromethamine | 1.32386 | (1) | TROMETHAMINE | 0.50 |
| Water, demineralized | | | AQUA (WATER) | ad |
| | | | | 100 |

| **C** | | | | |
|---|---|---|---|---|
| Carbopol^{®} Ultrez 21 | | (7) | ACRYLATES/C10-30 ALKYL ACRYLATE CROSSPOLYMER | 0.20 |
| Tegosoft^{®} TN | | (5) | C12-15 ALKYL BENZOATE | 0.40 |

| **D** | | | | |
|---|---|---|---|---|
| Preservatives | | | PARFUM | q.s. |
| Fragrance | | | | q.s. |

| **E** | | | | |
|---|---|---|---|---|
| Sodium Hydroxide, 10% | 1.05588 | (1) | AQUA (WATER), SODIUM HYDROXIDE | 0.50 |

### Procedure:

Disolve RonaCare^{®} Tromethamine in the water of phase B; add Eusolex^{®} 232 and stir until a clear solution is obtained. Then add the remaining ingredients of phase B. Heat up phase A1 and B to 65-70°C. Disperse phase A2 into phase A1. Add phase A into phase B while stirring.

Homogenize. Add phase C at 50-60°C while stirring. Homogenize. At 45°C add phase D. Adjust pH value with phase E to 6.8-7.2.

### Suppliers:

| | |
|---|---|
| (1) Merck KGaA, Darmstadt, Germany/EMD Performance Materials | (2) BASF AG |
| (3) 3V Sigma | (4) DSM Nutritional Products GmbH |
| (5) Evonik Nutrition & Care GmbH | (6) Biesterfeld |
| (7) Gattefossé (Deutschland) GmbH | |

### Example 12: Soft cream O/W lightening formulation

| **Ingredients** | **Art. No.** | **INCI (CTFA)** | | **[%]** |
|---|---|---|---|---|
| **A** | | | | |
| Eusolex^{®} OCR | 1.05377 (1) | OCTOCRYLENE | | 10.00 |
| Eusolex^{®} 9020 | 1.05844 | (1) | BUTYL METHOXYDIBENZOYLMETHANE (AVOBENZONE) | 2.00 |
| RonaCare^{®} AP | 1.30163 | (1) | BIS-ETHYLHEXYL HYDROXYDIMETHOXY BENZYLMALONATE | 2.00 |
| Oxynex^{®} K liquid | 1.08324 | (1) | PEG-8, TOCOPHEROL, ASCORBYL PALMITATE, ASCORBIC ACID, CITRIC ACID | 0.10 |
| Arlacel^{™} 165 | | (2) | GLYCERYL STEARATE, PEG-100 STEARATE | 3.00 |
| Span 60 | | (2) | SORBITAN STEARATE | 2.00 |
| Parteck^{®} LUB STA 50 | 1.00661 | (1) | STEARIC ACID | 2.00 |
| Floraesters 60 | | (3) | JOJOBA ESTERS | 1.00 |
| Shea Butter | | (4) | BUTYROSPERMUM PARKII BUTTER (BUTYROSPERMUM PARKII (SHEA) BUTTER) | 3.50 |
| Tegosoft^{®} TN | | (5) | C12-15 ALKYL BENZOATE | 5.00 |
| Miglyol^{®} 812 N | | (6) | CAPRYLIC/CAPRIC TRIGLYCERIDE | 3.00 |
| Avocado Oil | | (7) | PERSEA GRATISSIMA OIL (PERSEA GRATISSIMA (AVOCADO) OIL) | 3.00 |
| Xiameter^{®} PMX-0345 | | (8) | CYCLOPENTASILOXANE, CYCLOHEXASILOXANE | 5.00 |
| Xiameter^{®} PMX-200 Silicone Fluid (100cs) | | (8) | DIMETHICONE | 0.50 |

| **B1** | | | | |
|---|---|---|---|---|
| **RonaCare^{®}** Ectoin | **1.30200** | (1) | **ECTOINE** | 0.30 |
| RonaCare^{®} Disodium EDTA | 1.32221 | (1) | DISODIUM EDTA | 0.10 |
| Glycerol 85% Water, demineralized | 1.04091 | (1) | GLYCERIN, AQUA (WATER) AQUA (WATER) | 5.00 ad 100 |

| **B2** | | | | |
|---|---|---|---|---|
| Keltrol^{®} CG-RD | | (9) | XANTHAN GUM | 0.20 |

| **C** | | | | |
|---|---|---|---|---|
| Emblica^{®} | 1.30165 | (1) | PHYLLANTHUS EMBLICA EXTRACT | 2.00 |
| RonaCare^{®} Luremin^{®} | 1.30204 | (1) | SORBITOL, DIHYDROXY METHYLCHROMONE | 2.00 |
| Water, demineralized | AQUA (WATER) | | | 5.00 |

| **D** | | | | |
|---|---|---|---|---|
| Preservatives | | | | q.s. |
| Fragrance | PARFUM | | | q.s. |

### Procedure:

Disperse phase B2 into phase B1 and stir until homogenous.

Heat phases A and B to 80°C. Add phase A to phase B slowly while stirring. Homogenize.

Cool to 40°C and add phase C and D while stirring. Adjust pH value 5.0-5.5 if necessary.

Suppliers:

| | | |
|---|---|---|
| (1) Merck KGaA, Darmstadt, Germany/EMD Performance Materials | (2) | Croda |
| (3) Lehmann & Voss & Co. | (4) | H. Erhard Wagner GmbH |
| (5) Evonik Nutrition & Care GmbH | (6) | IOI Oleo GmbH |
| (7) Gustav Heess GmbH | (8) | Biesterfeld |
| (9) RAHN GmbH | | |

### Example 13: Soft Cream - O/W formulation tanning formulation

| **Ingredients** | **Art. No.** | | **INCI (CTFA)** | **[%]** |
|---|---|---|---|---|
| **A** | | | | |
| Cutina GMS V | | (1) | GLYCERYL STEARATE | 3.50 |
| Lanette^{®} O | | (1) | CETEARYL ALCOHOL | 1.50 |
| Cetiol^{®} OE | | (1) | DICAPRYLYL ETHER | 5.00 |
| Myritol^{®} 331 | | (1) | COCOGLYCERIDES | 3.00 |
| Miglyol^{®} 812 N | | (2) | CAPRYLIC/CAPRIC TRIGLYCERIDE | 4.00 |
| Cegesoft VP | | (1) | OLUS OIL (EUPHORBIA CERIFERA (CANDELILLA) WAX, VEGETABLE OIL, HYDROGENATED | 2.00 |
| | | | VEGETABLE OIL), HYDROGENATED VEGETABLE OIL, CANDELILLA CERA | |
| Jojoba Oil, bio | | (3) | SIMMONDSIA CHINENSIS (SIMMONDSIA CHINENSIS (JOJOBA) OIL) | 3.00 |
| Argan oil, bio | | (3) | ARGANIA SPINOSA (ARGANIA SPINOSA KERNEL OIL), (ARGAN OIL) | 1.00 |

| **B** | | | | |
|---|---|---|---|---|
| **RonaCare^{®} Ectoin** | **1.30200** | **(4)** | **ECTOINE** | **0.30** |
| Glycerol, anhydrous | 1.04093 | (4) | GLYCERIN | 3.00 |
| Keltrol^{®} CG-RD | | (5) | XANTHAN GUM | 0.60 |
| Water, demineralized | | | AQUA (WATER) | ad 100 |

| **C** | | | | |
|---|---|---|---|---|
| Dihydroxyacetone | 1.10150 | (4) | DIHYDROXYACETONE | 3.00 |
| Water, demineralized | | | AQUA (WATER) | 6.00 |

| **D** | | | | |
|---|---|---|---|---|
| Fragrance | | | PARFUM | q.s. |
| Preservatives | | | | q.s. |

### Procedure:

Phase B: disperse Keltrol in water + glycerol. Combine phase A. Heat phase A and phase B separately to 75°C. Add phase A to phase B while stirring. Homogenize. Cool down while stirring and add Phase C at approx. 35°C. Finally add the ingredients of phase D.

### Suppliers:

| | |
|---|---|
| (1) BASF AG | (2) IOI Oleo GmbH |
| (3) Gustav Heess GmbH | (4) Merck KGaA, Darmstadt, Germany/EMD Performance Materials |
| (5) RAHN GmbH | |

### Example 14: Soft-cream O/W formulation with insect repellent

| **Ingredients** | **Art. No.** | | **INCI (CTFA)** | **[%]** |
|---|---|---|---|---|
| **A** | | | | |
| IR3535^{®} | 1.11887 | (1) | ETHYL BUTYLACETYLAMINOPROPIONATE | 12.50 |
| Montanov^{™} 14 | | (2) | MYRISTYL ALCOHOL, MYRISTYL GLUCOSIDE | 1.50 |
| Montanov 202 | | (2) | ARACHIDYL ALCOHOL, BEHENYL ALCOHOL, ARACHIDYL GLUCOSIDE | 3.00 |
| Cetyl Alcohol | 1.00989 | (1) | CETYL ALCOHOL | 1.50 |
| Crodamol ISIS-LQ-(MV) | | (3) | ISOSTEARYL ISOSTEARATE | 3.50 |
| Cetiol^{®} B | | (4) | DIBUTYL ADIPATE | 3.00 |

| **B** | | | | |
|---|---|---|---|---|
| Water, demineralized | | | AQUA (WATER) | ad 100 |
| **RonaCare^{®} Ectoin** | **1.30200** | **(1)** | **ECTOINE** | **0.30** |
| Carbopol^{®} ETD 2050 | | (5) | CARBOMER | 0.20 |

| **C** | | | | |
|---|---|---|---|---|
| Sodium Hydroxide, 10% | 1.05588 | (1) | AQUA (WATER), SODIUM HYDROXIDE | 0.60 |

| **D** | | | | |
|---|---|---|---|---|
| RonaCare^{®} Bisabolol nat. | 1.30170 | (1) | BISABOLOL | 0.50 |
| Preservatives | | | | q.s. |
| Fragrance | | | PARFUM | q.s. |

### Procedure:

Heat phase A to 80°C and phase B to 75°C. Add phase A to phase B while stirring. Homogenize. Adjust pH with phase C (6.0-7.0) and cool down while stirring. Finally add phase D.

### Suppliers:

| | | |
|---|---|---|
| (1) Merck KGaA, Darmstadt, Germany/EMD Performance Materials | (2) | Seppic |
| (3) Croda | (4) | BASF AG |
| (5) Gattefossé (Deutschland) GmbH | | |

### Example 15: W/Si formulation with Sun protection

| **Ingredients** | **Art. No.** | | **INCI (CTFA)** | **[%]** |
|---|---|---|---|---|
| **A** | | | | |
| RonaCare^{®} AP | 1.30163 | (1) | BIS-ETHYLHEXYL HYDROXYDIMETHOXY BENZYLMALONATE | 1.00 |
| Dow Corning 5200 | | (2) | LAURYL PEG/PPG-18/18 METHICONE | 2.00 |
| Xiameter^{®} PMX-0345 | | (2) | CYCLOPENTASILOXANE, CYCLOHEXASILOXANE | 5.00 |
| Dow Corning 9040 Silicone Elastomer Blend | | (2) | CYCLOPENTASILOXANE, DIMETHICONE CROSSPOLYMER | 10.00 |
| Tegosoft^{®} APM | | (3) | PPG-3 MYRISTYL ETHER | 0.50 |
| Paraffin highly liquid | 1.07174 | (1) | PARAFFINUM LIQUIDUM (MINERAL OIL) | 10.00 |

| **B** | | | | |
|---|---|---|---|---|
| Eusolex^{®} T-S | 1.05334 | (1) | TITANIUM DIOXIDE (NANO), ALUMINA, STEARIC ACID | 10.00 |

| **C** | | | | |
|---|---|---|---|---|
| **RonaCare^{®} Ectoin** | **1.30200** | **(1)** | **ECTOINE** | **0.30** |
| RonaCare^{®} Sodium Chloride | 1.32260 | (1) | SODIUM CHLORIDE | 2.00 |
| Preservatives Water, demineralized | | | AQUA (WATER) | q.s. ad 100 |

| **D** | | | | |
|---|---|---|---|---|
| Fragrance | | | PARFUM | q.s. |

### Procedure:

COLD PROCESSING: Mix phase A with high energy and incorporate phase B thoroughly. Add pre-dissolved phase C slowly while stirring. Homogenize. Add Phase D while stirring.

### Suppliers:

| | |
|---|---|
| (1) Merck KGaA, Darmstadt, Germany/EMD Performance Materials | (2) Biesterfeld |
| (3) Evonik Industries AG | |

### Example 16: Rinse-off mask

| **Ingredients** | **Art. No.** | | **INCI (CTFA)** | **[%]** |
|---|---|---|---|---|
| **A** | | | | |
| **RonaCare^{®} Ectoin** | **1.30200** | **(1)** | **ECTOINE** | **2.00** |

| **B** | | | | |
|---|---|---|---|---|
| Yellow Illite Clay | | (2) | ILLITE | 50.00 |
| Tapioca Pure | | | TAPIOCA STARCH | 41.00 |
| Jojoba Oil, bio | | (3) | SIMMONDSIA CHINENSIS (SIMMONDSIA CHINENSIS (JOJOBA) OIL) | 3.00 |

| **C** | | | | |
|---|---|---|---|---|
| Ronastar^{®} Golden | 1.17042 | (1) | CALCIUM ALUMINUM | 4.00 |
| Sparks | | | BOROSILICATE, SILICA, CI 77891 (TITANIUM DIOXIDE), TIN OXIDE | |

### Procedure:

Put Yellow Illite Clay and Tapioca Pure in a blender and mix 2 times for 10 seconds. Add Jojoba Oil and mix 2 times for 10 seconds. Add RonaCare^{®} Renoumer and mix 2 times for 10 seconds. add phase C and mix 2 times for 10 seconds.

### Suppliers:

| | | |
|---|---|---|
| (1) Merck KGaA, Darmstadt, Germany/EMD Performance Materials | (2) | Natural Sourcing |
| (3) Gustav Heess GmbH | | |

## Claims

1. Ectoine for use for improving skin conditions of keratosis pilaris of a mammal.

2. Ectoine for use according to claim 1, **characterized in that** the mammal is a human.

## Patentansprüche

1. Ectoin zur Verwendung bei der Verbesserung der Hautbedingungen von Keratosis pilaris bei einem Säuger.

2. Ectoin zur Verwendung nach Anspruch 1, **dadurch gekennzeichnet, dass** es sich bei dem Säuger um einen Menschen handelt.

## Revendications

1. Ectoïne pour une utilisation destinée à l'amélioration de conditions cutanées de kératose pilaire chez un mammifère.

2. Ectoïne pour une utilisation selon la revendication 1, **caractérisée en ce que** le mammifère est un être humain.
